# EUROPÄISCHE PATENTANMELDUNG

(11) **EP 2 769 680 A1**
(43) Veröffentlichungstag der Anmeldung: **27.08.2014**
(21) Anmeldenummer: 13195274.9
(22) Anmeldetag: 02.12.2013
(51) Int. Cl.: A61B 17/00, A61F 2/95, A61F 2/966, A61F 6/18

(54) **Freigabevorrichtung zum Lösen eines medizinischen Implantats von einer Einführvorrichtung sowie Einführvorrichtung mit einer Freigabevorrichtung**

(30) Priorität: 22.02.2013 US 201361767800 P
(71) Anmelder: Biotronik AG, 8180 Bülach (CH)
(72) Erfinder: Wesselmann, Matthias, 8455 Rüdlingen (CH); Quint, Bodo, 8154 Oberglatt (CH)
(74) Vertreter: Lindner-Vogt, Karin L.

(57) **Zusammenfassung**

Die Erfindung betrifft eine Freigabevorrichtung (160, 160a, 160b) zum Lösen eines medizinischen Implantats (155) von einer Einführvorrichtung (120), bei welcher das Implantat (155) durch eine Relativbewegung zwischen einem ersten und einem zweiten Einführelement (140, 145) freisetzbar ist, umfassend einen Körper (10, 10a, 10b) mit einem proximalen Ende (12), das im Benutzungszustand von einem distalen Ende (130) der Einführvorrichtung (120) entfernt ist, und einem distalen Ende (14), das im Benutzungszustand dem distalen Ende (130) der Einführvorrichtung (120) zugewandt ist, wobei zwischen dem proximalen und dem distalen Ende (12, 14) ein erstes Griffsegment (34, 34'), ein zweites Griffsegment (34, 34') und zumindest ein Aktor (26) vorgesehen ist, wobei der zumindest eine Aktor (26) manuell bedienbar und im Wesentlichen axial verschieblich im Wesentlichen in zumindest das erste Griffsegment (34) bringbar ist, um eine gezielte Relativbewegung in Längsrichtung (22) zwischen dem ersten und dem zweiten Einführelement (140, 145) der Einführvorrichtung (120) zu bewirken.
Die Erfindung betrifft ferner eine Einführvorrichtung (120) mit einer solchen Freigabevorrichtung (160, 160a, 160b).

## Beschreibung

Die Erfindung betrifft eine Freigabevorrichtung zum Lösen eines medizinischen Implantats von einem Katheter und einen Katheter mit einer Freigabevorrichtung zum Freigeben eines medizinischen Implantats zur Implantation in einen tierischen und/oder menschlichen Körper nach den Oberbegriffen der unabhängigen Patentansprüche.

In der Medizin kommen häufig Implantate zum Einsatz, die zur Erfüllung von Ersatzfunktionen permanent oder zumindest für einen längeren Zeitraum in einen tierischen und/oder menschlichen Körper eingebracht werden. Zu nennen wären hier beispielsweise Herzschrittmacher, Hirnschrittmacher für Parkinsonpatienten, Herzimplantate, wie Herzklappen oder so genannte Septum-closure devices, Cochleaimplantate, Retina Implantate, zahnmedizinische Implantate, Implantate zum Gelenkersatz, Gefäßprotesen z. B einsetzbar in die Pulmunarvene, Okkluder, beispielsweise für den Appendix, oder Stents.

Implantate sind zum Einführen in den Körper mit Kathetern verbunden und müssen am Einsatzort genau platziert und definiert freigegeben werden können. Hierzu ist beispielsweise aus US 6,709,667 B1 bekannt, das Implantat durch eine Schiebbewegung freizugeben.

Der Erfindung liegt die Aufgabe zugrunde, eine Freigabevorrichtung anzugeben, mit der das gezielte Freigeben eines Implantats verbessert ist.

Eine weitere Aufgabe ist in der Bereitstellung einer entsprechenden Einführvorrichtung zu sehen.

Die Aufgabe wird erfindungsgemäß durch die Merkmale der unabhängigen Ansprüche gelöst. Günstige Ausgestaltungen und Vorteile der Erfindung ergeben sich aus den weiteren Ansprüchen und der Beschreibung.

Es wird eine Freigabevorrichtung zum Lösen eines medizinischen Implantats von einer Einführvorrichtung vorgeschlagen, bei welcher das Implantat durch eine Relativbewegung zwischen einem ersten und einem zweiten Einführelement freisetzbar ist. Die Freigabevorrichtung umfasst einen Körper, mit einem proximalen Ende, das im Benutzungszustand von einem distalen Ende der Einführvorrichtung entfernt ist, und einem distalen Ende, das im Benutzungszustand dem distalen Ende der Einführvorrichtung zugewandt ist, wobei zwischen dem proximalen und dem distalen Ende ein erstes Griffsegment, ein zweites Griffsegment und zumindest ein Aktor vorgesehen ist, wobei der zumindest eine Aktor manuell bedienbar und im Wesentlichen axial verschieblich im Wesentlichen in das zumindest erste Griffsegment bringbar ist, um eine gezielte Relativbewegung in Längsrichtung zwischen dem ersten und dem zweiten Einführelement der Einführvorrichtung zu bewirken.

Durch die erfindungsgemäße Ausgestaltung kann eine Freigabevorrichtung bereitgestellt werden, die intuitiv und einfach bedienbar gestaltet ist. Ferner kann diese komfortabel und sicher von einem Bediener, wie einem Arzt, gehalten, manipuliert und bearbeitet werden. Zudem kann eine hohe Freisetzkraft bei geringer, insbesondere manueller, Bedienkraft eingeleitet werden. Ferner können mittels des erfindungsgemäßen Aktors kraftvolle Bewegungen übertragen werden, durch die das Implantat zuverlässig und homogen freigegeben bzw. präzise und vorsichtig positioniert werden kann. Somit kann sowohl eine kraftvolle und /oder eine fein dosierte, vorsichtige Freisetzung ermöglicht werden. Zudem wird so ein einfaches Konzept zu einer Freigabe des Implantats verwirklicht. Besonders vorteilhaft kann mittels der erfindungsgemäßen Ausgestaltungen eine Repositionierung oder ein Rückzug des teilweise freigegebenen Implantats, beispielsweise bei Fehlpositionierung, ermöglicht werden. Dies ist insbesondere der Fall, da gemäß der erfindungsgemäßen Vorrichtung besonders große Kräfte zum wieder Zusammendrücken des teilweise expandierten Implantats erzeugt werden können. Des Weiteren weist die Freigabevorrichtung eine einfache Handhabung und Montage des Implantats auf der Einführvorrichtung, beispielsweise einem Katheter, im Vorbereitungslabor auf. Auch kann durch das simple Design ein Herstellungsaufwand reduziert werden, wodurch zudem Herstellungskosten gering gehalten werden können.

In diesem Zusammenhang soll unter einem "Griffsegment" ein Teil der Freigabevorrichtung verstanden werden, der von einer und/oder mehreren Händen des Bedieners direkt bzw. mittelbar angreifbar ist, um die Freigabevorrichtung zu bewegen, zu manipulieren und/oder dergleichen. Jedes der Griffsegmente kann vom jedem, dem Fachmann für einsetzbar erachteten Element gebildet sein, wie beispielsweise von dem Gehäuse des Körpers, einem Teil des Gehäuses oder von einem zusätzlich angeordneten Griff, wie einem Bügelgriff und einem Pistolengriff. Ferner soll unter einem "Aktor" eine Struktur oder ein Element erstanden werden, die/das eine vom Bediener ausgeführte Aktion auf zumindest einen Teil der Freigabevorrichtung, wie beispielsweise eines der Einführelemente, insbesondere zu einer Freisetzung oder Abdeckung des Implantats unmittelbar und/oder mittelbar überträgt. Im folgenden Text wird der zumindest eine Aktor als der Aktor bezeichnet.

Der Aktor kann von jedem, dem Fachmann für einsetzbar erachteten Element gebildet sein, wie einem Stift, einem Bolzen, einem Knopf, einem Drehknopf, einem Drückknopf, einem Schieber oder einem Hebel. Ferner ist es vorteilhaft, wenn der Aktor von einem Bedienhebel gebildet ist, wodurch der Aktor besonders robust und langlebig gestaltet werden kann. Zudem kann der Aktor konstruktiv einfach und komfortabel von außerhalb eines Gehäuses des Körpers bedient werden. Mit einer solchen Ausgestaltung können bedienerfreundlich große Kräfte übertragen werden. Zudem kann gegenüber Systemen des Standes der Technik, die mit einem kleinen Bedienrad arbeiten, größere Angriffsflächen zur Verfügung gestellt werden. Zudem kann über eine individuell voreinstellbare Hebellänge des Bedienhebels unter Ausnutzung des Hebelgesetzes die Kraft auf das zumindest eine Einführelement bzw. den Außenschaft verstärkt und gleichzeitig auch der Betrag von Freisetzschritte festgelegt werden (siehe unten). Eine bevorzugte Orientierung oder Ausrichtung des Aktors kann insbesondere eine im Wesentlichen senkrecht zu einer Längsachse des Körpers darstellen, wodurch eine raumsparende Anordnung und eine intuitive Bedienung möglich ist.

Unter "manuell bedienbar" soll hier verstanden werden, dass der Aktor unmittelbar und direkt, insbesondere händisch, von dem Bediener angefasst und/oder bedient werden kann. "Im Wesentlichen axial verschieblich" soll hier bedeuten, dass eine Abweichung einer Verschiebebewegung des Aktors von bis zu 10° von einer axialen Richtung als axiale Verschiebung verstanden werden soll. Ferner soll unter der Wendung "im Wesentlichen in zumindest das erste Griffsegment bringbar" verstanden werden, dass der Aktor bevorzugt zu zumindest 50%, vorteilhaft zu zumindest 75% und besonders bevorzugt zu zumindest 95% in zumindest das erste Griffsegment einführbar und/oder versenkbar ist. Dies ist insbesondere bezogen auf eine axiale Breite des Aktors.

Eine gezielte Relativbewegung in Längsrichtung stellt hier insbesondere eine Bewegung in axialer und/oder longitudinaler Richtung dar. Unter dem Begriff "bewirken" soll hier und im weiteren Text "erzeugen, veranlassen und/oder erreichen" verstanden werden.

Bevorzugt ist eine Ausgangsposition des Aktors eine senkrechte Anordnung des Aktors zu der Richtung zumindest eines der Einführelemente oder eine zu höchstens 5° bis 10° verkippte Anordnung zur senkrechten Anordnung des Aktors. Der Begriff "kippbar" soll als "schwenkbar" oder "schwingbar" verstanden werden. Es wäre zudem auch denkbar zusätzlich zu der im Wesentlichen axialen Verschiebung des Aktors eine Dreh- und/oder Kippbewegung des Aktors in seinem Lagepunkt vorzusehen. Der Aktor kann somit in Folge seiner Bewegung auch schräg zu der axialen bzw. longitudinalen Richtung angeordnet werden. Zusätzlich könnte auch eine im Wesentlichen vertikale Bewegung des Aktors vorteilhaft sein. Der Definition von "im Wesentlichen vertikal" ist analog zu oben ausgeführter Definition von "im Wesentlichen axial" zu verstehen.

Ferner ist es vorteilhaft, wenn eine Drehachse des ersten Griffsegments im Wesentlichen senkrecht zu zumindest einem der Einführelemente verläuft. Bei solch einer Anordnung kann eine Drehbewegung des ersten Griffsegments für den Bediener im Wesentlichen behinderungsfrei durchgeführt werden. Gemäß einer bevorzugten Realisierung kann auch das erste Griffsegment im Wesentlichen senkrecht zu zumindest einem der Einführelemente verlaufen. Hierdurch kann die Freigabevorrichtung mit einer natürlichen und unverkrampften Handhaltung für den Bediener ermüdungsfrei gehalten werden. In diesem Zusammenhang soll unter "im Wesentlichen senkrecht" eine Abweichung der Richtung der Drehachse bzw. des ersten Griffsegments zu der Richtung zumindest eines der Einführelemente zur senkrechten Anordnung von bis zu 30° verstanden werden. In einer bevorzugten Realisierung und wie oben erwähnt kann das erste Griffsegment drehbar ausgeführt sein, und insbesondere um eine Drehachse im Wesentlichen senkrecht zu der Längsachse des Körpers orientiert ist. Bevorzugterweise ist das erste Griffsegment von einem Pistolenhandgriff gebildet. Damit kann ein besonders ergonomisches Design bereitgestellt werden, durch das das erste Griffsegment mit einer ganzen Hand umgriffen werden kann. Dies ist besonders bedienerfreundlich, da es für den Bediener ermüdungsfrei erfolgen kann.

Des Weiteren wird vorgeschlagen, dass das zweite Griffsegment im Wesentlichen koaxial zu zumindest einem der Einführelemente verläuft, insbesondere dem Teil des zumindest einen Einführelements bzw. beider Einführelemente, der innerhalb des Körpers angeordnet ist. Dadurch kann eine Manipulation der Lage der Freigabevorrichtung besonders zuverlässig auf zumindest eines der Einführelemente übertragen werden. Unter "im Wesentlichen koaxial" soll eine Anordnung des zweiten Griffsegments verstanden werden, die höchstens 5° bis 10° zur axialen Anordnung des Griffsegments abweicht. Des Weiteren wird vorgeschlagen, dass das zweite Griffsegment einstückig mit einem Gehäuse des Körpers bzw. der Freigabevorrichtung ausgeführt ist, wodurch eine große Angriffsfläche für die Handhabung der Freigabevorrichtung geschaffen ist. Hierbei soll unter "einstückig" verstanden werden, dass das zweite Griffsegment und das Gehäuse von demselben Bauteil gebildet sind und/oder nur unter Funktionsverlust zumindest eines der Bauteile voneinander getrennt werden können.

Eine Übertragung der Bewegung des Aktors auf zumindest eines der Einführelemente bzw. das äußere Einführelement kann mittels jedem, dem Fachmann für sinnvoll erscheinendem Konzept erfolgen, wie beispielsweise durch eine stoffschlüssige Verbindung zwischen dem Aktor und zumindest einem der Einführelemente bzw. dem äußeren Einführelement oder durch einen Formschluss und/oder Kraftschluss zwischen dem Aktor und einem der Einführelemente bzw. dem äußeren Einführelement oder einem mit dem Einführelement verbindbaren weiteren Element und/oder Bauteil, wie etwa einem Übertragungselement. In letzterem Fall stellt die Verbindung zwischen dem Aktor und einem der Einführelemente eine mittelbare bzw. durch das weitere Element und/oder Bauteil vermittelte Verbindung dar.

Bei der vorliegend Erfindung kann davon ausgegangen werden, dass zwischen dem äußeren Einführelement und dem Körper so viel Reibung herrscht, dass das äußere Einführelement bei der Rückbewegung des Aktors und/oder des Übertragungselements durch ein Federelement (siehe unten) fixiert an seiner Position verbleibt bzw. sich nicht mit dem Aktor und/oder dem Übertragungselement bewegt. Dadurch kann die gezielte Relativbewegung in Längsrichtung zwischen dem ersten und dem zweiten Einführelement vorteilhaft in feinen Freisetzschritten erfolgen. Dies erfolgt durch mehrmaliges Betätigen des Aktors zeitlich nacheinander.

In einer bevorzugten Realisierung ist vorgesehen, dass die gezielte Relativbewegung in Längsrichtung zwischen dem ersten und dem zweiten Einführelement der Einführvorrichtung zumindest mittels eines Kraftschlusses zwischen dem Aktor und einem der Einführelemente erfolgt. Durch den Kraftschluss kann ein bewährtes und robustes Prinzip der Kraftübertragung verwendet werden. Hierbei soll unter der Wendung "mittels eines Kraftschlusses zwischen dem Aktor und einem der Einführelemente" sowohl ein unmittelbarer Kraftschluss wie eine mittelbarer Kraftschluss, vermittelt über das weitere Element und/oder Bauteil und/oder das Übertragungselement, zwischen dem Aktor und einem der Einführelemente verstanden werden. Ein Element, welches die Vorschubbewegung über einen Kraftschluss auf eines der Einführelement überträgt, wie insbesondere der Aktor oder ein Übertragungselement, ist aus zumindest einem sehr harten und/oder steifen Material, wie z.B. Inox AISI 316L oder Inox AISI 306, gebildet. Das Einführelement stellt bevorzugt das äußere Einführelement dar. Der Kraftschluss kann durch den Aktor und/oder durch eine axial und/oder vertikal versetzte und/oder gekippte Position des Aktors konstruktiv einfach erreicht und auf das zumindest eine Einführelement bzw. den Außenschaft übertragen werden.

Bevorzugterweise erfolgt die gezielte Relativbewegung in Längsrichtung zwischen dem ersten und dem zweiten Einführelement der Einführvorrichtung zumindest mittels eines lösbaren Kraftschlusses, zwischen dem Aktor und einem der Einführelemente bzw. dem äußeren Einführelement. Hierdurch kann die etappenartige oder stufenweise Freisetzung des Implantats vorteilhaft einfach realisiert werden.

Alternativ und/oder zusätzlich erfolgt die gezielte Relativbewegung in Längsrichtung zwischen dem ersten und dem zweiten Einführelement der Einführvorrichtung zumindest mittels eines Formschlusses zwischen dem Aktor und einem der Einführelemente. Hierzu kann vorzugsweise ein Material des zumindest einen Einführelements entsprechend angepasst werden und weist beispielsweise ein Material mit hoher Haftreibung auf. Hierdurch kann das zumindest eine Einführelement relativ zum Aktor im Kippzustand in Position gehalten werden. Das Material kann jedes, vom Fachmann für sinnvoll erachtetes Material mit einer hohen Haftreibung sein.

Es wird zudem vorgeschlagen, dass die Freigabevorrichtung ein Stellelement aufweist, mit dem ein Betriebsmodus einstellbar ist, wodurch ein Modiwechsel einfach erfolgen kann. Das Stellelement kann von jedem, dem Fachmann für anwendbar erachtetem Element gebildet sein, wie einem Drückknopf, einem Hebel, einem Drehknopf oder einem Stellschieber. Ferner kann es vorteilhaft sein, wenn die Freigabevorrichtung ein Stellelement aufweist, mit dem ein Betriebsmodus anzeigbar ist. Hierdurch können Montageaufwand, Platz und Kostend sparend zwei Funktionen in einem Bauteil vereint werden. Es wird zudem vorgeschlagen, dass die Freigabevorrichtung ein Anzeigeelement zu einer Anzeige eines eingestellten Betriebsmodus aufweist. Das Anzeigenelement kann von jedem, dem Fachmann für sinnvoll erachtetem Element gebildet sein, wie einem Display, einer Skalierung, einem Textfeld oder einer Bauteilorientierung. Unter einem Anzeigenelement soll auch eine Einheit von mehreren gleichartigen oder unterschiedlichen Anzeigeelementen verstanden werden. Vorteilhafter weise sind das Stellelement und das Anzeigenelement einstückig miteinander ausgeführt, wodurch eine kompakte Vorrichtung erreicht werden kann. Hierbei soll unter "einstückig" verstanden werden, dass das Stellelement und das Anzeigenelement von demselben Bauteil gebildet sind und/oder nur unter Funktionsverlust zumindest eines der Bauteile voneinander getrennt werden können.

Gemäß einer vorteilhaften Ausgestaltung kann für einen Wechsel zwischen zumindest zwei Betriebsmodi das Stellelement ein Wirkelement aufweisen, wodurch der Betriebsmodus leicht bestimmt werden kann. Das Wirkelement kann von jedem, dem Fachmann für anwendbar erachtetem Element gebildet sein, wie einer Feder, einem Hebel, einem Schiebschalter oder insbesondere von einem exzentrischen Element. Eine Wirkung des Wirkelements kann besonders einfach erhalten werden, wenn es exzentrisch zu einer Drehachse des Stellelements angeordnet ist. Gemäß der erfindungsgemäßen Ausgestaltung kann die Betätigung des Stellelements, insbesondere in der Ausführung als drehbares Griffsegment dessen Drehung, konstruktiv einfach verschiedene Reaktionen an unterschiedlichen Bauteilen oder Konstellationen von unterschiedlichen Bauteilen, wie beispielsweise dem Aktor oder einer Ausnehmung des ersten Griffsegments oder deren Anordnung zueinander, vermitteln. Hierbei ist die Drehachse insbesondere im Wesentlichen senkrecht zur Längsrichtung des zumindest einen Einführelements und einer Erstreckung des Aktors ausgerichtet. Bevorzugt ist das Wirkelement einstückig mit dem Stellelement verbunden oder in diesem ausgeformt. Der Definition von "einstückig" ist analog zu oben ausführt zu verstehen.

Vorteilhafterweise ist das erste Griffsegment einstückig mit dem Stellelement ausgeführt, wodurch die Bedienung des Stellelements Prozess straffend und ohne Zeitverzögerung ausgelöst werden kann. Gemäß einer vorteilhaften Ausgestaltung ist das Anzeigeelement bevorzugt einstückig mit einem drehbaren Griffsegment ausgeführt. Hierdurch kann vorteilhaft Platz gespart werden. Das drehbare Griffsegment ist bevorzugt das erste Griffsegment. Der Definition von "einstückig" ist jeweils analog zu oben ausführt zu verstehen. Sind das drehbare Griffsegment, das Anzeigeelement und das Stellelement in einem Bauteil vereint kann der Bediener besonders komfortabel und schnelle einen Betriebsmodus einstellen und auch erfassen. Bevorzugt ist das drehbare Griffsegment von einem unsymmetrisch ausgeführten Bauteil gebildet, wobei der Betriebsmodus und bevorzugt auch die Bewegungsrichtung, beispielsweise des äußeren Einführelements, durch die Orientierung des Griffsegments, insbesondere für den Bediener intuitiv, angezeigt werden kann.

Des Weiteren wird vorgeschlagen, dass die Freigabevorrichtung zumindest ein im Wesentlichen axial verschiebliches Übertragungselement umfasst, wodurch eine Übertragung der Bewegung des Aktors konstruktiv einfach erfolgen kann. Zudem können die unterschiedlichen Bauteile, wie der Aktor und das Übertragungselement dadurch vorteilhaft aus unterschiedlichen Materialien gefertigt sein, die speziell auf eine Funktion oder eine Belastung des Bauteils abgestimmt sind. Bezüglich der Definition von "im Wesentlichen axial verschieblich" wird auf die Definition oben verwiesen. Das "im Wesentlichen axial verschiebliche Übertragungselement" wird im folgenden Text als "das Übertragungselement" benannt. Für eine schnelle und sichere Übertragung überträgt das Übertragungselement die axiale Bewegung des Aktors vorteilhafterweise beim Betätigen des Aktors auf zumindest eines der Einführelemente und insbesondere das äußere Einführelement, um die gezielte Relativbewegung in Längsrichtung zwischen dem ersten und dem zweiten Einführelement der Einführvorrichtung zu bewirken.

Des Weiteren kann es vorteilhaft sein, wen das Übertragungselement einstückig mit dem äußeren Einführelement ausgeführt ist. Dadurch kann die Verbindung dieser Bauteile verliersicher und zuverlässig gewährleistet werden.

Eine alternative und bevorzugte Weiterbildung besteht darin, dass das Übertragungselement als ein separates Bauteil zu dem äußeren Einführelement ausgeführt ist, wodurch auch hier Materialien der Bauteile individuell auf ihre Funktion etc. ausgelegt werden können. Die Übertragung der Bewegung des Aktors auf das Übertragungselement kann hier vorteilhaft mittels eine lösbaren Verbindung übermittelt werden. Auch hier kann dies mittels jedem, dem Fachmann für anwendbar erscheinendem Konzept, wie beispielsweise einem Formschluss, einem Kraftschluss und/oder einem Reibschluss, erfolgen.

Auch für eine Verbindung zwischen dem Aktor und dem Übertragungselement wäre jede, dem Fachmann für passend erachtete, bspw. oben genannte, Verbindungsart denkbar. Demgemäß kann der Aktor beispielsweise kraft- und/oder formschlüssig an/in dem Übertragungselement angeordnet sei. Ferner wäre es denkbar, dass der Aktor und/oder das Übertragungselement beispielsweise mit einer Beschichtung versehen sein könnte, die eine hohe Haftreibung vermittelt. Ein Material einer solchen Beschichtung kann jedes, vom Fachmann für sinnvoll erachtetes Material sein, wie insbesondere ein Polymer und insbesondere ein Material ausgewählt aus der Gruppe bestehend aus Polyamid, Polyester, Polyether-Block-Amid, Silikon, Polyurethan. Hierdurch kann der Klemmkörper insbesondere mit geringem Gewicht ausgeführt werden. Eine besonders zuverlässige Positionierung des Implantats in der Einführvorrichtung kann wegen seiner hohen Haftreibung vorteilhaft erreicht werden, wenn das Material z.B. ein Polyether-Block-Amid wie PEBAX der Firma Arkema ist. Hierbei sind alle Härtegrade einsetzbar.

In einer weiteren möglichen Realisierung weist das Übertragungselement zumindest einen ersten Anschlag für den Aktor auf. Hierdurch kann eine Bewegung des Aktors einfach auf das Übertragungselement und eines der Einführelemente bzw. das äußere Einführelement übertragen werden. Bevorzugt weist das Übertragungselement zumindest einen zweiten Anschlag für den Aktor auf, wobei der erste und der zumindest zweite Anschlag bevorzugt axial an unterschiedliche Seiten des Aktors angeordnet sind, wodurch diese entgegengesetzte Bewegungen des Aktors limitieren bzw. übertragen können.

Das Übertragungselement kann hier von jedem, dem Fachmann für sinnvoll erscheinendem Element gebildet sein, wie einem Stift, einem Bolzen, einer Platte, einer Scheibe, einem Ring oder einer Buchse. In einer bevorzugten Ausgestaltung weist das zumindest eine Übertragungselement eine zylindrische Buchse auf, wodurch ein robustes und zuverlässiges Bauteil verwendet werden kann. Ferner ist das Übertragungselement somit auf eine Form der Einführelemente und insbesondere des äußeren Einführelement abgestimmt. Eine platzsparende Anordnung kann bereitgestellt werden, wenn die Buchse im Wesentlichen koaxial zu zumindest einem der Einführelemente bzw. dem äußeren Einführelement angeordnet ist.

Zudem kann es vorteilhaft sein, wenn das Übertragungselement zumindest eine Durchführung für zumindest eines der Einführelemente und insbesondere eines äußeren

Einführelements und/oder von einem Außenschaft aufweist. Dies erlaubt eine kompakte Anordnung und eine konstruktiv einfache Möglichkeit zum Erreichen eines Wirkschlusses zwischen dem Übertragungselement und zumindest einem der Einführelemente. In diesem Zusammenhang soll unter einem Wirkschluss insbesondere ein Formschluss und/oder ein Kraftschluss verstanden werden.

Das Übertragungselement kann beispielweise auch so ausgebildet sein, dass die im Wesentlichen axiale Bewegung des Aktors eine Kippbewegung des Übertragungselements auslöst. Dadurch wird dieses auf dem äußeren Einführelement verkantet und das äußere Einführelement kann durch diesen Kraftschluss oder diese Klemmwirkung mit dem Übertragungselement konstruktiv einfach axial bewegt werden. Das Übertragungselement könnte in diesem Fall beispielsweise als ein sich in Bewegungsrichtung jeweils konisch verbreiternde Buchse ausgebildet sein. Ist zusätzlich zu der axialen Bewegung des Aktors ein Schwenken bzw. eine Kippbewegung des Aktors vorgesehen kann ein mögliches Kippen des Übertragungselements einfach, beispielsweise durch einen ortsfesten Eingriff des Aktors in das Übertragungselement, realisiert werden. Bei einer kippbaren Ausgestaltung des Übertragungselements und/oder des Aktors stellt die koaxiale Position des Übertragungselements und/oder eine senkrechte Position des Aktors in Bezug auf zumindest eines der Einführelemente eine kontaktlose Position dar, die gekippte bzw. schräge Position hingegen eine Kontaktposition.

Prinzipiell könnte das Übertragungselement aber auch so ausgeführt sein, dass sich dessen radialer Durchmesser vermittelt durch die axiale Verschiebung des Aktors verringert und dadurch beispielsweise das Einführelement gequetscht wird. Dies wäre konstruktiv beispielsweise durch eine zusätzliche vertikale Bewegung des Aktors realisierbar.

Gemäß einer weiteren Ausgestaltung ist vorgesehen, dass das Übertragungselement zumindest einen Kraftübertragungsbereich aufweist, wodurch diese Bereich in Bezug auf zum Beispiel seiner Dimension(en), seines Materials, seiner Form etc. speziell auf seine Funktion der Kraftübertragung des Kraftschlusses zwischen dem Aktor und dem einen der Einführelemente angepasst werden kann. Der Kraftübertragungsbereich ist insbesondere in Kontakt mit dem Aktor bringbar oder ständig mit diesem in Kontakt.

Ferner weist das Übertragungselement zumindest einen Gleitbereich auf, wobei bevorzugt der zumindest eine Gleitbereich eine Gleitbewegung des Übertragungselements auf einem der Einführelemente vermittelt. Hierdurch kann eine Bewegung des Übertragungselements relativ zum (äußeren) Einführelement besonders homogen und mit reduzierter Reibung erfolgen, was insbesondere bei einem Modus einer schnellen Freigabe des Implantats vorteilhaft ist. Es wäre hier denkbar entweder einen kompletten Teil des Übertragungselements aus einem Material mit guten Gleiteigenschaften auszustatten oder auch nur auf den Gleitbereich eine Beschichtung aus einem solchen Material aufzubringen. Als ein solches Material wäre jedes, von Fachmann für einsetzbar erachtetes Material denkbar, wie beispielsweise Teflon, Graphit oder dergleichen. In einer bevorzugten Realisierung wird der Kraftübertragungsbereich von zwei Gleitbereichen axial flankiert, wodurch eine Gleitbewegung besonders gleichmäßig erfolgen kann. Diese beiden Gleitbereiche können gleich ausgeführt sein oder sich in mindestens einem Parameter, wie Dimension (axiale Länge, radiale Dicke), Kontur, Form, Material, Beschichtung usw., unterscheiden.

Gemäß einer bevorzugten und besonders vorteilhaften Ausführungsform der Erfindung umfasst das Übertragungselement zumindest einen Übergangsbereich, wodurch dieser Bereich für spezielle Funktionen vorgesehen sein kann. Der Übergangsbereich ist bevorzugt axial zwischen dem zumindest einen Kraftübertragungsbereich und dem zumindest einen Gleitbereich angeordnet. Hierdurch können diese Bereiche bezogen auf ihre Eigenschaften unabhängig voneinander ausgestaltet werden. Der Übergangsbereich kann aber auch eine eigene spezielle Ausgestaltung aufweisen und dadurch eine bestimmte Funktion übernehmen. Beispielsweise können der Übergangsbereich und der Kraftübertragungsbereich und/oder der Gleitbereich jeweils eine radiale Materialstärke aufweisen, wobei insbesondere die Materialstärke des Übergangsbereichs dünner ist als die Materialstärke des Kraftübertragungsbereichs und/oder des Gleitbereichs. Hierdurch weist der Übergangsbereich im Vergleich zum Kraftübertragungsbereich und/oder zum Gleitbereich eine reduzierte Biegesteifigkeit auf. Somit kann es ermöglicht werden, dass beispielsweise der Kraftübertragungsbereich des Übertragungselements eine andere Orientierung oder Position, wie eine gekippte bzw. verkantete oder ein vertikale Position, als der Gleitbereich des Übertragungselements einnehmen kann. Grundsätzlich könnte dies auch durch ein anderes bzw. weicheres Material des Übergangsbereichs im Vergleich zu dem Kraftübertragungsbereichs und/oder dem Gleitbereich gewährleistet werden.

In einer weiteren Ausgestaltung der Erfindung wird vorgeschlagen, dass das Übertragungselement mit zumindest einem Federelement vorgespannt ist, wodurch das Übertragungselement konstruktiv einfach in einer eingestellten Position gehalten werden kann. Unter einem Federelement soll hier jedes federnde und/oder elastische Element, und insbesondere eine Feder, beispielsweise in der Form einer Druckfeder, verstanden werden. Ferner wird vorgeschlagen, dass das Übertragungselement durch das Federelement in seine Ausgangsposition zurückstellbar ist. Durch diese vorteilhafte Realisierung kann auf ein zusätzliches von einem Bediener bedienbares Rückstellmittel für das Federelement und/oder das Übertragungselement verzichtet werden, wodurch Montageaufwand, Bauraum und Kosten eingespart werden können. Zudem kann mit der bevorzugten Ausgestaltung des Federelements als eine Rückstellfeder ein zuverlässiges Bauteil mit einem geringen Gewicht verwendet werden. Hierbei wird davon ausgegangen, dass zwischen dem äußeren Einführelement und dem Körper so viel Reibung herrscht, dass das äußere Einführelement bei der Rückbewegung das zumindest eine Übertragungselement durch das Federelement fixiert an seiner Position verbleibt bzw. sich nicht mit dem zumindest einen Übertragungselement bewegt. Um dies auf jeden Fall zu verhindern, kann zusätzlich ein Blockierelement vorgesehen sein, dass das äußere Einführelement in Position hält. Dieses Blockierelement kann von jedem, dem Fachmann für nutzbar erachtetem Element gebildet sein, wie beispielsweise eine Fläche mit hoher Reibung oder eine Blockierscheibe, die beispielsweise radial um das äußere Einführelement gelegt ist.

Eine vielseitig und flexibel einsetzbare Freigabevorrichtung kann vorteilhaft bereitgestellt werden, wenn ein Betriebsmodus der schnellen Freigabe des Implantats vorgesehen ist. Konstruktiv einfach kann dieser Betriebsmodus eingestellt werden, wenn für die schnelle Freigabe des Implantats das Übertragungselement relativ zu zumindest einem der Einführelemente im Wesentlichen koaxial stellbar ist und/oder in einer koaxialen Position gehalten wird. Bezüglich der Definition von "im Wesentlichen koaxial" wird auf die Ausführungen oben verwiesen. In der koaxialen Anordnung ist der Kraftschluss zwischen dem Aktor und/oder dem Übertragungselement und dem zumindest einen Einführelement bzw. dem Außenschaft aufgelöst bzw. besteht keine Kraftschluss zwischen dem Aktor und/oder dem Übertragungselement und dem zumindest einen Einführelement bzw. dem Außenschaft.

Die axiale Bewegungsrichtung des Aktors weist vorteilhafterweise eine gleiche Richtung wie die Bewegung des äußeren Einführelements auf, wodurch die Bedienung des Aktors für den Bediener besonders intuitiv und leicht verständlich ist. Alternativ kann es auch dieselbe Richtung sein. Ferner wird vorgeschlagen, dass eine axiale Bewegung des Aktors in Richtung des proximalen Endes des Körpers eine Freigabe des Implantats vermittelt. Hierdurch ist der Wirkmechanismus für den Bediener intuitiv verständlich In einer weiteren Ausgestaltung der Erfindung wird vorgeschlagen, dass eine axiale Bewegung des Aktors in Richtung des distalen Endes des Körpers eine Abdeckung des Implantats mit einem der Einführelemente vermittelt, wodurch auch diese Bewegungsvermittlung für den Bediener leicht erfassbar ist. Mittels der unterschiedlichen Richtungen für das Freigeben und das Abdecken des Implantats können die beiden Hauptfunktionen der Freigabevorrichtung Fehler reduzierend vermittelt werden. Hierbei soll unter einer Abdeckung des Implantats ein Bedecken des Implantats mit insbesondere dem äußeren Einführelement verstanden werden.

Eine bevorzugte Weiterbildung besteht darin, dass der Körper zumindest eine Aufnahme für ein inneres Einführelement zu einer unbeweglichen Fixierung des inneren Einführelements am Körper aufweist. Hierdurch kann das innere Einführelement bzw. ein Innenschaft sicher am Körper gehalten bzw. verankert werden. Die Aufnahme kann von jeder, dem Fachmann für sinnvoll erachteten Struktur gebildet sein, wie beispielsweise einem Haken, einem Bolzen, einer Ausnehmung, einer Vertiefung, einem Spalt oder einem Schlitz. Zudem wird vorgeschlagen, dass die Aufnahme am äußeren Einführelement bzw. am Außenschaft ausgebildet ist, wodurch die Verankerung besonders direkt und unmittelbar gestaltet ist. Hierfür muss das äußere Einführelement bzw. der Außenschaft so ausgelegt sein, dass er Zug- und Druckkräfte aufnehmen kann ohne zu knicken. Eine besonders gute Fixierung kann vorteilhaft erreicht werden, wenn die Aufnahme als ein Schlitz ausgeführt ist. Alternativ und/oder zusätzlich ist der innere Außenschaft in einer weiteren Verankerung am Körper und/oder dessen Gehäuse fixiert. Hierdurch kann das äußere Einführelement entlastet werden. Hierfür kann jede, dem Fachmann für sinnvoll erachtete Verbindungsart, wie ein Kraft-, ein Form- oder ein Stoffschluss, beispielsweise mittels Schweißen, Löten, Schrauben, Nageln, Crimpen oder Kleben, in Frage kommen

In einer weiteren Ausgestaltung der Erfindung wird vorgeschlagen, dass der Körper zumindest eine Durchführung für zumindest eines der Einführelemente aufweist. Dies erlaubt eine kompakte Anordnung, welche das durchgeführte Einführelement stabilisiert und schützt. Ist die Einführvorrichtung ein Katheter, so kann das betreffende Einführelement ein äußeres Einführelement des Katheters sein.

Gemäß einer zusätzlichen Realisierung wird vorgeschlagen, dass ein aus dem Körper ragendes Einführelement, wie insbesondere das am proximalen Ende des Körpers aus diesem hinausragende äußere Einführelement, zumindest eine Markierung aufweist, die dazu vorgesehen ist anzuzeigen, wie viel vom Implantat freigesetzt wurde. Hierdurch kann ein Fortschritt der Freigabe des Implantats besonders einfach überwacht werden. Eine solche Markierung kann von jedem, dem Fachmann für sinnvoll erachtetem Mittel gebildet sein, wie beispielsweise einer Zahl, einem Buchstaben, einem Strich, einer Einkerbung, einem Farbcode oder dergleichen. Vorteilhafterweise sind eine Vielzahl von Markierungen vorhanden. Zusätzlich ist es vorteilhaft, wenn die Markierung dazu vorgesehen ist anzuzeigen, ob das Implantat in die Freigabevorrichtung zurückziehbar ist. Hierdurch kann eine Blockierung des Implantats in der Freigabevorrichtung vorteilhaft verhindert und dadurch eine Fehlfunktion der Freigabevorrichtung vermieden werden.

Gemäß einem weiteren Aspekt der Erfindung wird eine Einführvorrichtung zum Einführen des medizinischen Implantats vorgeschlagen, welches durch eine Relativbewegung zwischen dem ersten und dem zweiten Einführelement freisetzbar ist, umfassend die Freigabevorrichtung zum Lösen des medizinisches Implantats, umfassend den Körper, mit dem proximalen Ende, das im Benutzungszustand von dem distalen Ende der Einführvorrichtung entfernt ist, und dem distalen Ende, das im Benutzungszustand dem distalen Ende der Einführvorrichtung zugewandt ist, wobei zwischen dem proximalen und dem distalen Ende ein erstes Griffsegment, ein zweites Griffsegment und zumindest ein Aktor vorgesehen ist, wobei der zumindest eine Aktor manuell bedienbar und im Wesentlichen axial verschieblich im Wesentlichen in zumindest das erste Griffsegment bringbar ist, um eine gezielte Relativbewegung in Längsrichtung zwischen dem ersten und dem zweiten Einführelement der Einführvorrichtung zu bewirken.

Durch die erfindungsgemäße Ausgestaltung kann eine Einführvorrichtung bereitgestellt werden, die intuitiv und einfach bedient werden kann. Ferner kann diese komfortabel und sicher von einem Bediener, wie einem Arzt, gehalten, manipuliert und bearbeitet werden. Zudem kann eine hohe Freisetzkraft bei geringer, insbesondere manueller, Bedienkraft eingeleitet werden, wodurch zudem kraftvolle Bewegungen übertragen werden können, durch die das Implantat zuverlässig und homogen freigegeben bzw. präzise und vorsichtig positioniert werden kann. Somit kann sowohl eine kraftvolle und /oder eine fein dosierte, vorsichtige Freisetzung ermöglicht werden. Besonders vorteilhaft kann mittels der erfindungsgemäßen Ausgestaltungen eine Repositionierung oder ein Rückzug des teilweise freigegebenen Implantats, beispielsweise bei Fehlpositionierung, ermöglicht werden. Des Weiteren weist die Einführvorrichtung eine einfache Handhabung und Montage des Implantats im Vorbereitungslabor auf. Auch kann durch das simple Design ein Herstellungsaufwand reduziert werden, wodurch zudem Herstellungskosten gering gehalten werden können.

Gemäß einer vorteilhaften Ausgestaltung kann das Implantat ein selbstexpandierendes Implantat sein, wodurch es sich bei der Freigabe selbsttätig öffnen kann. Durch das selbstexpandierende Implantat kann ein zusätzliches Expandiermittel entfallen. Dadurch können vorteilhaft Raum und Montageaufwand für dieses eingespart werden. Hierdurch kann auch die Einführvorrichtung weniger komplex gestaltet werden. Grundsätzlich wäre es jedoch auch möglich ein ballonexpandierbares Implantat zu verwenden. Hierfür müsste die Einführvorrichtung jedoch entsprechend angepasst werden, was der Fachmann aufgrund seiner Fachkenntnis selbstständig löst.

Die Erfindung ist nachfolgend beispielhaft, anhand von in Zeichnungen dargestellten Ausführungsbeispielen, näher erläutert. Es zeigen in schematischer Darstellung:
- Fig. 1: einen Schnitt durch ein günstiges Ausführungsbeispiel einer Einführvorrichtung und einer Freigabevorrichtung mit einem montierten Implantat;
- Fig. 2: einen Schnitt durch die Freigabevorrichtung der Fig. 1 entlang der Linie II-II;
- Fig. 3A: die Freigabevorrichtung der Fig. 1 vorbereitet für eine Freigabe des Implantats;
- Fig. 3B: eine Seitenansicht eines Griffsegments der Freigabevorrichtung der Fig. 1 mit Anzeige des Betriebsmodus der langsamen Freigabe des Implantats;
- Fig. 4: die Freigabevorrichtung der Fig. 1 während der langsamen Freigabe des Implantats;
- Fig. 5A: die Freigabevorrichtung der Fig. 1 vorbereitet für eine Abdeckung des Implantats;
- Fig. 5B: eine Seitenansicht des Griffsegments der Freigabevorrichtung der Fig. 1 mit Anzeige des Betriebsmodus der Abdeckung des Implantats;
- Fig. 6: die Freigabevorrichtung der Fig. 1 während der Abdeckung des Implantats;
- Fig. 7A: die Freigabevorrichtung der Fig. 1 vorbereitet für eine schnelle Freigabe des Implantats;
- Fig. 7B: eine Seitenansicht des Griffsegments der Freigabevorrichtung der Fig. 1 mit Anzeige des Betriebsmodus der schnellen Freigabe des Implantats;
- Fig. 8: eine alternative Freigabevorrichtung vorbereitet für eine langsame Freigabe eines Implantats;
- Fig. 9: die Freigabevorrichtung der Fig. 8 während der langsamen Freigabe des Implantats;
- Fig. 10: die Freigabevorrichtung der Fig. 8 vorbereitet für eine Abdeckung des Implantats;
- Fig. 11: die Freigabevorrichtung der Fig. 8 während der Abdeckung des Implantats und;
- Fig. 12: eine weitere alternative Freigabevorrichtung während der langsamen Freigabe des Implantats.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

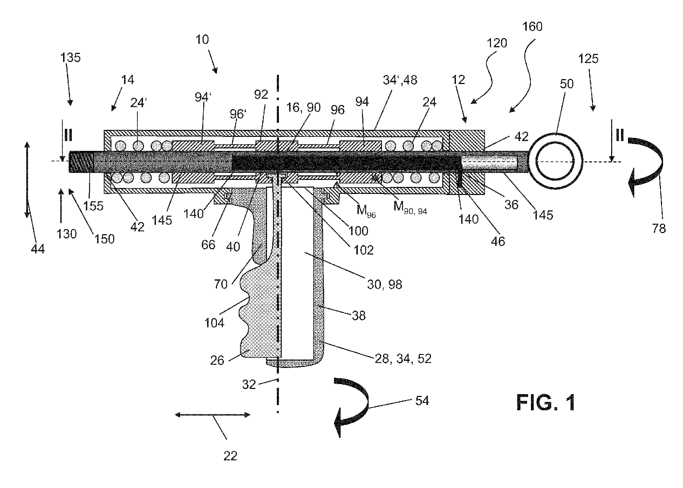

Fig. 1 zeigt einen Längsschnitt durch ein günstiges Ausführungsbeispiel einer Freigabevorrichtung 160 einer Einführvorrichtung 120. Die Einführvorrichtung 120 ist beispielsweise ein Katheter mit einem Schaftbereich 135 mit zwei koaxial angeordneten Einführelementen 140, 145, z.B. einem Innenschaft (Einführelement 140) und einem diesen umgebenden Außenschaft (Einführelement 145), welcher wiederum von einer hier nicht dargestellten Außenhülle umgeben sein kann. Die Einführvorrichtung 120 ist in Anwenderbetrieb, also während einer Befestigung des Implantats 155 an der Freigabevorrichtung 160 oder während der Implantation mit ihrem proximalen Ende 125 einem Bediener zugewandt. Das Implantat 155 ist am distalen Ende 130 des Schaftbereichs 135 zwischen Innenschaft und Außenschaft platziert und soll am Implantationsort im tierischen oder menschlichen Körper freigegeben werden.

Die Freigabevorrichtung 160 dient zum Lösen des medizinischen Implantats 155 von der Einführvorrichtung 120. Das Implantat 155 ist an dem vom Bediener abgewandten Ende 130 des Schaftbereichs 135 beispielsweise in der Nähe einer hier nicht im Detail gezeigten Katheterspitze 150 angeordnet. Das Implantat 155 ist beispielsweise um das innere Einführelement 140 gelegt (nicht im Detail gezeigt) und wird durch eine Relativbewegung zwischen dem ersten und dem zweiten Einführelement 140, 145 freigesetzt. Hierbei ist das innere Einführelement 140 mit der Katheterspitze 150 verbunden, das äußere Einführelement 145 hingegen nicht.

Die Freigabevorrichtung 160 umfasst einen Körper 10 mit einem proximalen Ende 12, das im Benutzungszustand von dem distalen Ende 130 der Einführvorrichtung 120 entfernt ist, und mit einem distalen Ende 14, das im Benutzungszustand dem distalen Ende 130 der Einführvorrichtung 120 zugewandt ist. Der Körper 10 bzw. das äußere Einführelement 145 weist eine Aufnahme 36, in der Form eines axial verlaufenden Schlitzes, für ein proximales Ende des inneren Einführelements 140 zu einer unbeweglichen Fixierung des inneren Einführelements 140 am Körper 10 auf. Zudem ist das proximale Ende des inneren Einführelement 140 in radialer Richtung 44 nach der Aufnahme 36 des äußeren Einführelements 145 in einer Verankerung 46 eines Gehäuses 48 des Körpers 10, beispielsweise form-, kraft- oder stoffschlüssig, gehalten. Zudem weist der Körper 10 mehrere Durchführungen 42 für das äußere Einführelement 145 auf, wodurch dieses am proximalen Ende 12 des Körpers 10 aus diesem austritt. An einem proximalen Ende des äußeren Einführelements 145 ist außerhalb des Körpers 10 ein Bedienelement 50 in der Form eines Schlaufengriffs ausgeformt.

Die Freigabevorrichtung 160 bzw. der Körper 10 weist zwischen dem proximalen und dem distalen Ende 12, 14 ein erstes Griffsegment 34 und ein zweites Griffsegment 34' auf. Das erste Griffsegment 34 ist in einer axial mittigen Position des Körpers 10 angeordnet und verläuft im Wesentlichen senkrecht zu beiden Einführelementen 140, 145 bzw. ist im Wesentlichen senkrecht zum Körper 10 ausgerichtet. In einer Arbeitssituation, wie beispielsweise während der Implantation, weist das erste Griffsegment 34 relativ zum Körper 10 nach unten. Zudem ist das erste Griffsegment 34 als ein Pistolengriff 52 ausgebildet. Ferner ist das erste Griffsegment 34 so ausgeführt bzw. über eine axial auskragende Scheibe 66 im Gehäuse 48 gelagert, dass es um seine Drehachse 32 in Umfangsrichtung 54 des ersten Griffsegments 34 gedreht werden kann. Die Drehachse 32 des ersten Griffsegments 34 verläuft im Wesentlichen senkrecht zu den Einführelementen 140, 145. Das zweite Griffsegment 34' hingegen ist einstückig mit dem Gehäuse 48 ausgeführt und verläuft somit im Wesentlichen koaxial zu beiden Einführelementen 140, 145.

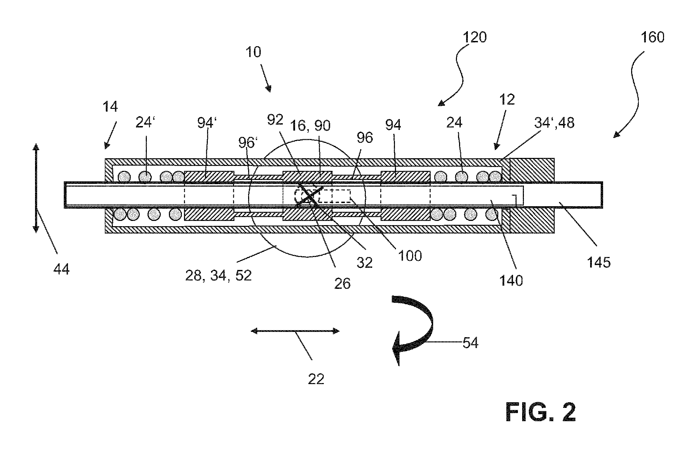

Zudem ist am ersten Griffsegment 34 ein Aktor 26 angeordnet, der vom Bediener manuell bedienbar und im Wesentlichen axial verschieblich in das ersten Griffsegment 34 bringbar ist, um eine gezielte Relativbewegung in Längsrichtung 22 zwischen dem ersten und dem zweiten Einführelement 140, 145 der Einführvorrichtung 120 zu bewirken (Details siehe unten). Der Aktor 26 ist als ein Bedienhebel ausgeführt. Zudem ist der Aktor 26 in seinem unaktuierten Zustand zum Teil in einer Ausnehmung 98 des ersten Griffsegments 34 angeordnet und erstreckt sich im Wesentlichen senkrecht zu der Drehachse 32 des ersten Griffsegments 34. Eine Bedienoberfläche 104 bzw. eine Angriffsfläche des Aktors 26 ist außerhalb der Ausnehmung 98 angeordnet. Des Weiteren erstreckt sich der Aktor 26 durch einen sich axial erstreckenden Schlitz 100 in dem Gehäuse 48 des Körpers 10 (siehe Fig. 2) und greift in eine Aufnahme 102, die in einem Übertragungselement 16 ausgeformt ist.

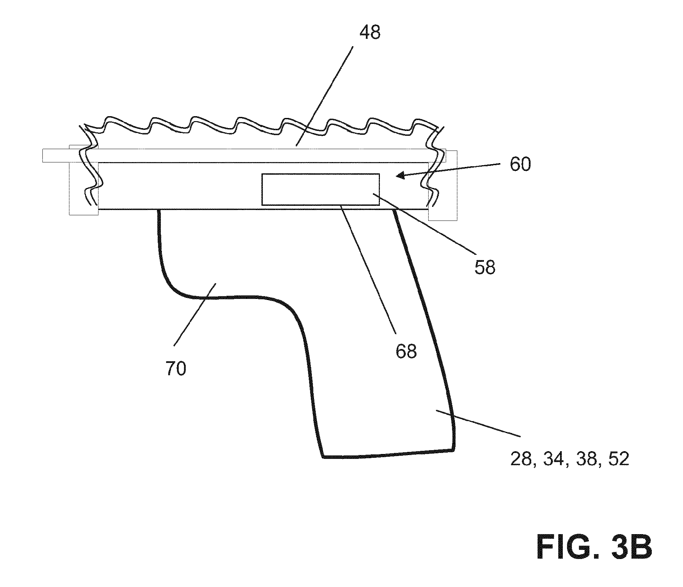

Ferner weist die Freigabevorrichtung 160 ein Stellelement 28 auf, mit dem ein Betriebsmodus der Freigabevorrichtung 160 einstellbar ist (siehe unten). Hierbei ist das Stellelement 28 einstückig mit dem ersten Griffsegment 34 bzw. dem Pistolengriff 52 ausgeführt. Zudem weist die Freigabevorrichtung 160 ein Anzeigeelement 38 zu einer Anzeige des eingestellten Betriebsmodus auf. Auch das Anzeigeelement 38 ist einstückig mit dem drehbaren Griffsegment 34 bzw. dem Pistolengriff 52 ausgeführt und ist je Betriebsmodus von einem den Betriebsmodus bezeichnenden Schriftzug 58 gebildet, der je nach eingestelltem Betriebsmodus in eine Display 60 des Gehäuses 48 des Körpers 10 sichtbar wird (vgl. Fig. 3B, 5B, 7B).

Zwischen dem proximalen und dem distalen Ende 12, 14 des Körpers 10 weist dieser das Übertragungselement 16 auf, welches im Wesentlichen axial verschieblich ist und die axiale Bewegung des Aktors 26 beim Betätigen des Aktors 26 auf das äußere Einführelemente 145 überträgt, um die gezielte Relativbewegung in Längsrichtung 22 zwischen dem ersten und dem zweiten Einführelement 140, 145 der Einführvorrichtung 120 zu bewirken. Das Übertragungselement 16 ist als ein Kraftübertragungsbereich 90 einer zylindrischen Buchse 92 ausgeführt, welche im Wesentlichen koaxial zu beiden Einführelementen 140, 145 angeordnet ist bzw. sich in einer Umfangsrichtung 78 des Körpers 10 entlang eines Umfang des äußeren Einführelements 145 erstreckt. Somit weist das Übertragungselement 16 eine Durchführung 40 für das äußere Einführelement 145 auf. Ferner weist das Kraftübertragungsbereich 90 zur Übertragung der Bewegung des Aktors 26 die Aufnahme 102 auf. Die Buchse 92 ist zur Übertragung der Bewegung des Aktors 26 verliersicher mit dem äußeren Einführelement 145 verbunden. Hierbei kann jede kraft-, form- und/oder stoffschlüssige Verbindung zwischen der Buchse 92 und den äußeren Einführelement 145 in Frage kommen.

Bevorzugt sind das äußere Einführelement 145 und das Kraftübertragungsbereich 90 und damit der Aktor 26 über einen lösbaren Kraftschluss verbunden. Hierfür weist die Freigabevorrichtung 160 ein Mittel zum Herstellen und Lösen des Kraftschlusses, wie beispielsweise ein hier nicht näher gezeigte Klemmmittel, auf. Dieses kommt bei einer stufenweisen Freisetzung oder Abdeckung des Implantats 155 zum Einsatz (siehe unten).

Zudem weist die zylindrische Buchse 92 zwei Endbereiche 94, 94' auf, wobei einer der Endbereiche 94 an einem Ende der Buchse 92 angeordnet ist, das in Richtung des proximalen Endes 12 des Körpers weist und der andere Endbereich 94' an einem Ende der Buchse 92, das in Richtung des distalen Endes 14 des Körpers weist. Beide Endbereiche 94, 94' sind über einen Übergangsbereich 96, 96' mit dem Kraftübertragungsbereich 90 verbunden. Der Kraftübertragungsbereich 90 und die Endbereiche 94, 94' weisen jeweils im Wesentlichen eine gleiche Materialstärke M₉₀, M₉₄ in radialer Richtung 44 der Buchse 92 auf. Die Übergangsbereiche 96, 96' hingegen weist eine radiale Materialstärke M₉₆ auf, die dünner ist als die Materialstärken M₉₀, M₉₄ des Kraftübertragungsbereichs 90 und der Endbereiche 94, 94'.

Das Übertragungselement 16 ist mit zwei Federelementen 24, 24' vorgespannt, die beide als eine Druckfeder ausgeführt sind. Das Federelement 24 erstreckt sich zwischen dem proximalen Ende 12 des Körpers 10 und dem Endbereich 94 des Übertragungselements 16 und das andere Federelement 24'erstreckt sich zwischen dem distalen Ende 14 des Körpers 10 und dem Ende 94' des Übertragungselements 16. In einer beispielsweise vor der Implantation eingestellten Ausgangkonfiguration ist das Übertragungselement 16 von jedem Federelement 24, 24' vorgespannt und zwischen den Federelementen 24, 24' in einer axial mittigen Position im Gehäuse 48 des Körpers 10 axial fixiert.

Mit dem Aktor 26 können zwei unterschiedliche Betriebsmodi vermittelt werden und zwar eine Freigabe des Implantats 155 und eine Abdeckung des Implantats 155 mit dem äußeren Einführelement 145. Zu einem Wechsel zwischen den beiden Betriebsmodi weist das Stellelement 28 ein Wirkelement 30 auf. Dieses Wirkelement 30 ist von der Ausnehmung 98 des ersten Griffsegments 34 gebildet und ist exzentrisch zu der Drehachse 32 des Stellelements 28 angeordnet. Hierdurch ist auch der Aktor 26 exzentrisch in der Ausnehmung 98 angeordnet. Bei unaktuiertem Zustand des Aktors 26 ist in dieser Stellung das Übertragungselement 16 bzw. die Buchse 92 in seiner/ihrer axial mittigen Position, bei der beide Federelemente 24, 24' gleich vorgespannt sind.

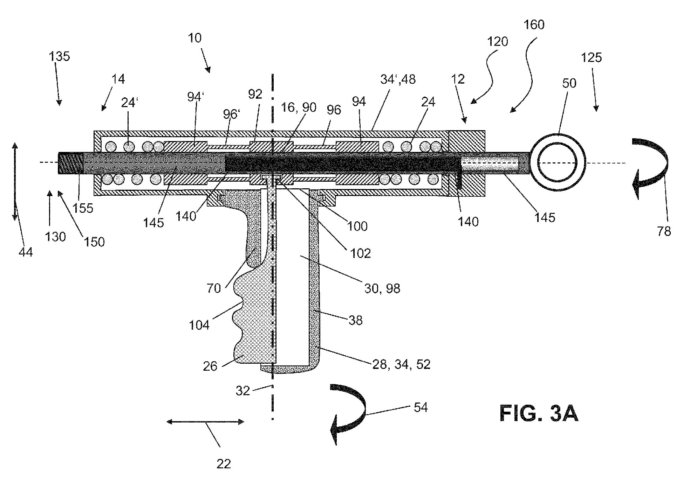

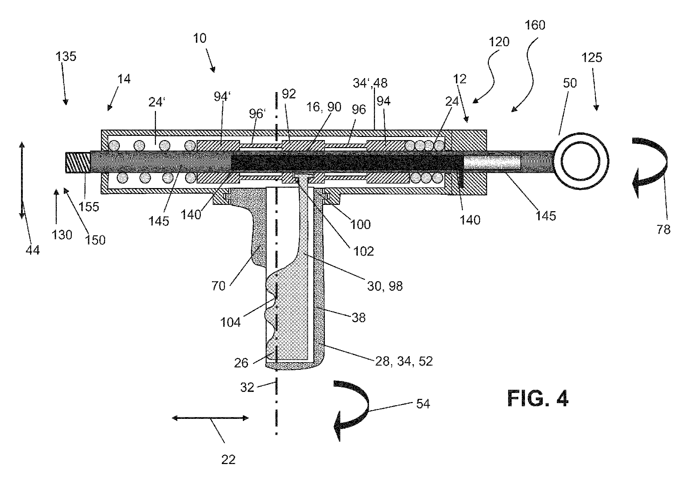

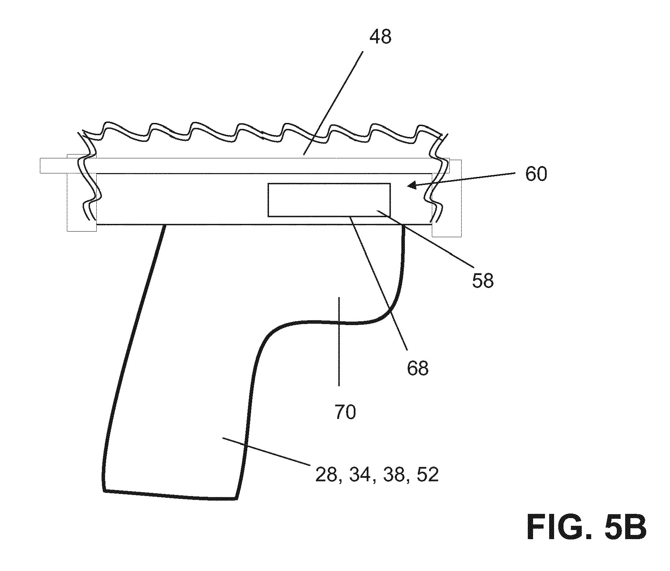

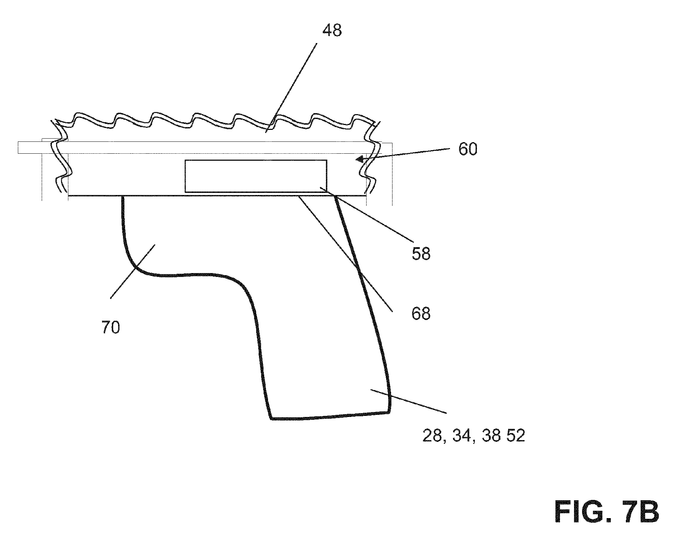

Anhand der Fig. 3A, 3B und 4 wird nun eine langsame Freigabe des Implantats 155 anhand eines so genannten Deploy-Modus, beschrieben. Zu der Implantation des Implantats 155 im Körper, wird die so vorbereitete Einführvorrichtung 120 in den Körper eingeführt (nicht gezeigt). Zum Freisetzen des Implantats 155, wird das Stellelement 28 bzw. das erste Griffsegment 34 (Pistolengriff 52) in Umfangsrichtung 54 gedreht bis im Display 60 des Anzeigeelements 38 der Schriftzug 58 "DEPLOY" erscheint (vgl. Fig. 3B, hier sowie in den Fig. 5B und 7B ist der Aktor 26 nicht dargestellt). Dieses kann auch der voreingestellte Betriebsmodus sein. Der Schriftzug 58 "DEPLOY" kann zum Beispiel in einem hier nicht näher dargestellten Rad eingraviert sein, welches das erste Griffsegment 34 drehbar lagert. Ein Fenster 68 im Gehäuse 48 lässt je nach Griffsegmentstellung den entsprechenden Schriftzug 58 auf dem Rad erscheinen. Der Pistolengriff 52 ist auch dadurch als Anzeigenelement 38 ausgeführt, da er durch eine Orientierung seines Knaufes 70 zur Anlage eines hier nicht gezeigten Zeigefingers indirekt die Bewegungsrichtung des äußeren Einführelements 145 anzeigt. Das heißt, um das Implantat 155 freizusetzen wird der Außenschaft in Richtung des proximalen Endes 12 bewegt werden und der Knauf 70 des ersten Griffsegments 34 in Richtung des distalen Endes 14 zeigen. Auch die Bedienoberfläche 104 des Aktors 26 weist in die Richtung des distalen Endes 14. Das exzentrische Wirkelement 30 bzw. die Ausnehmung 98 ist hier so angeordnet, dass in Richtung des proximalen Endes 12 ein Freiraum zum Versenken des Aktors 26 bereitgestellt wird. In dieser Einstellung ist die Buchse 92 in ihrer mittleren Position im Körper 10 und die Federelemente 24, 24' sind im Wesentlichen wenig gestaucht.

Hier sind das Kraftübertragungsbereichs 90 und das äußere Einführelement 145 kraftschlüssig miteinander verbunden. Um den Kraftschluss herzustellen wird das Klemmmittel bedient. Dieses ist beispielsweise als ein Quetschring oder ein Druckschalter ausgeführt, welcher beispielsweise einen Durchmesser des Kraftübertragungsbereichs 90 verringert oder dessen runde Kontur zu eine unrunden Kontur verändert (nicht im Detail gezeigt). Bei einer Ausgestaltung als ein Druckschalter würde dieser zu einer Betätigung durch den Bediener von außerhalb des Körpers 10 in das Gehäuse 48 eintreten und kann bei seiner Betätigung das Übertragungsbereich 90 radial kontaktieren. Zudem bewegt er sich bevorzugt mit dem Übertragungsbereich 90 bei der Bewegung in Längsrichtung 22 und wird dabei von einem axial verlaufenden Schlitz im Gehäuse 48 geführt. Bei einer konstruktiv einfachen Ausgestaltung wäre der Druckschalter beispielsweise radial gegenüber des Aktors 26 angeordnet und würde auf den Anteil des Übertragungselements 16 drücken, der radial gegenüber der Aufnahme 102 des Aktors 26 angeordnet ist. Durch die geringere Materialstärke M₉₆ der Übergangsbereiche 96, 96' und dadurch deren geringerer Biegesteifigkeit im Vergleich zu den Materialstärken M₉₀, ₉₄ des Kraftübertragungsbereichs 90 und der Endbereiche 94, 94'erfolgt der Kraftschluss nur zwischen dem Kraftübertragungsbereich 90 der Buchse 92 und dem äußeren Einführelement 145 und nicht zwischen den Endbereichen 94, 94' der Buchse 92 und dem äußeren Einführelement 145.

Bei einem Betätigen des Aktors 26 bzw. des Bedienhebels in diesem Betriebsmodus wird dieser axial in Richtung des proximalen Endes 12 verschoben. Hierdurch bewegt er sich komplett in die Ausnehmung 98 des ersten Griffsegments 34 (siehe Fig. 4). Durch den Eingriff des Aktors 26 in die Aufnahme 102 des Kraftübertragungsbereichs 90 wird auch der Kraftübertragungsbereich 90 parallel zu der Längsrichtung 22 in Richtung des proximalen Endes 12 bewegt. Wegen der verliersicheren Verbindung zwischen der Buchse 92 und dem äußeren Einführelement 145 wird auch dieses in Richtung des proximalen Endes 12 geschoben. Dies erfolgt bis das Federelement 24 völlig gestaucht ist was die Bewegung des Aktors 26 limitiert.

Zur Bewegung des Übertragungselements 16 in seine Ausgangsposition muss nun der Kraftschluss wischen diesem und dem äußeren Einführelement 145 gelöst werden. Dies erfolgt mittels Lösen des Klemmmittels. Wird nun der Aktor 26 losgelassen kann das Federelement 24, das auch eine Rückstellfeder ist, expandieren und drückt die Buchse 92 mit dem Kraftübertragungsbereich 90 und dem Aktor 26 in Längsrichtung 22 und in Richtung des distalen Endes 14 zurück in die mittlere Position bzw. die Ausgangsposition (vgl. Fig. 3). Die Endbereich 94, 94' der Buchse 92 sind als Gleitbereiche 94, 94' ausgeführt, um eine Gleitbewegung des Übertragungselements 16 auf dem äußeren Einführelement 145 zu vermitteln. Da bei der vorliegend Erfindung davon ausgegangen werden kann, dass zwischen dem äußeren Einführelement 145 und dem Körper 10 viel Reibung herrscht, verbleibt das äußere Einführelement 145 bei der Rückbewegung des Aktors 26 und des Übertragungselements 16 fixiert an seiner Position bzw. es bewegt sich nicht mit dem Aktor 26 und dem Übertragungselement 16 zurück. Damit kann der Vorgang des Freilegens des Implantats 155 solange wiederholt werden, bis das äußere Einführelement 145 das Implantat 155 völlig frei gelegt hat. Wie viel vom Implantat 155 freigesetzt wurde kann beispielsweise durch eine hier nicht näher dargestellte Markierung überwacht werden. Diese Markierung ist zum Beispiel an einem Ende des äußeren Einführelements 145 angebracht, welches am proximalen Ende 12 des Körpers 10 aus diesem herausragt. Sie kann zum Beispiel als eine Reihe aufsteigender Zahlen ausgebildet sein.

Somit vermittelt der Aktor 26 durch seine axiale Bewegung die gezielte Relativbewegung in Längsrichtung 22 zwischen dem ersten und dem zweiten Einführelement 140, 145 bzw. es resultiert durch die Betätigung des Aktors 26 ein Zurückziehen des Außenschafts relativ zum Innenschaft und damit auch zum Implantat 155. Hierdurch wird das Implantat 155 freigelegt und expandiert beispielsweise entweder bei der Ausführung als selbstexpandierbares Implantat 155, wie einer stentbasierten Herzklappe, selbstständig und/oder mit Hilfe eines Ballons. Im Anschluss wird die Freigabevorrichtung 160 bzw. der Innenschaft in den Außenschaft zurückgezogen und die Einführvorrichtung 120 aus dem Körper entfernt. Das Implantat 155 verbleibt vollständig positioniert im Körper (nicht gezeigt).

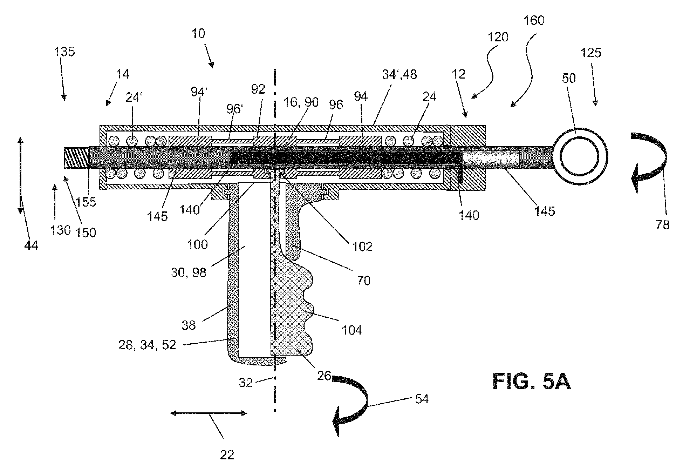

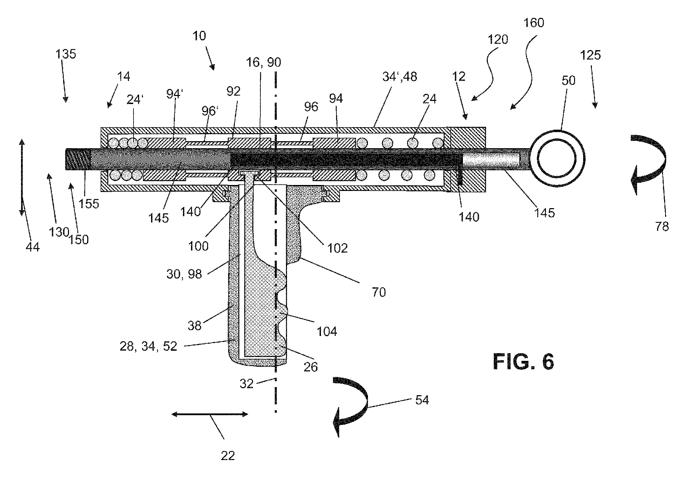

Anhand der Fig. 5A, 5B und 6 wird nun der Betriebsmodus der Abdeckung des Implantats 155 mit dem äußeren Einführelement 145, einem so genannten Retrieval-Modus, beschrieben. Dieser kann beispielsweise zu einer Montage des Implantats 155 in der Einführvorrichtung 120 oder zu einem Zurückziehen bzw. wieder Abdecken des Implantats 155 während der Implantation bei einer Fehlpositionierung oder -funktion des Implantats 155 verwendet werden. Die Kräfte, die hierzu nötig sind, sind wesentlich größer als bei der Freisetzung, da das beispielsweise teilentfaltete Implantat 155 wieder auf seinen gestauchten bzw. zusammengefalteten kleinen Durchmesser gebracht werden muss. Der Retrieval-Modus wird dadurch eingestellt, dass das Stellelement 28 bzw. das erste Griffsegment 34 (Pistolengriff 52) in Umfangsrichtung 54 bis im Display 60 des Anzeigeelements 38 der Schriftzug 58 "RETRIEVE" erscheint gedreht wird (vgl. Fig. 5B). Dadurch zeigt der Knauf 70, an dem nun eine nicht gezeigte Handfläche anliegt, und die Bedienoberfläche 104 in Richtung des proximalen Endes 12 um zu symbolisieren, dass das äußere Einführelement 145 in Richtung des distalen Endes 14 bewegt werden wird (vgl. Fig. 5A und 5B). In dieser Einstellung ist die Buchse 92 in ihrer mittleren Position im Körper 10 und die Federelemente 24, 24' sind im Wesentlichen wenig gestaucht.

Bei dieser Stellung des ersten Griffsegments 34 ist das exzentrische Wirkelement 30 bzw. die Ausnehmung 98 so angeordnet, dass in Richtung des distalen Endes 14 ein Freiraum zum Versenken des Aktors 26 bereitgestellt wird. Ist der Kraftschluss zwischen dem Kraftübertragungsbereich 90 und dem äußeren Einführelement 145 mittels des Klemmmittels geschlossen und wird nun der Aktor 26 bzw. der Bedienhebel in Richtung des distalen Endes 14 axial verschoben, tritt dieser komplett in die Ausnehmung 98 des ersten Griffsegments 34 ein (siehe Fig. 6). Hierdurch wird auch die Buchse 92 in Richtung des distalen Endes 14 bewegt und nimmt das äußere Einführelement 145 über die verliersicher Verbindung in Richtung des distalen Endes 14 mit. Dies erfolgt bis das Federelement 24' völlig gestaucht ist was die Bewegung des Aktors 26 limitiert. An der Katheterspitze 150 ergibt sich also ein Vorschieben des äußeren Einführelements 145 zur Katheterspitze 150 hin. Wird das Implantat 155 durch das innere Einführelement 140 axial blockiert, schiebt sich das äußere Einführelement 145 also (wieder) über das Implantat 155 und bedeckt dieses.

Wird nun nach dem Lösen des Kraftschlusses der Aktor 26 bzw. der Bedienhebel losgelassen, schiebt das Federelement 24', das auch eine Rückstellfeder darstellt, die Buchse 92 mit dem Kraftübertragungsbereich 90 und dem Aktor 26 in Richtung des proximalen Endes 12 bis in seine mittleren Position bzw. seiner Ausgangsposition zurück. Dieser Vorgang kann wiederholt werden, bis das äußere Einführelement 145 das Implantat 155 wieder komplett abdeckt. Ob das Implantat 155 in die Freigabevorrichtung 160 zurückziehbar ist kann mittels der Markierung am äußeren Einführelement 145 bestimmt werden. Als Resultat ist das Implantat 155 in dem äußeren Einführelement 145 platziert und die Montage ist abgeschlossen.

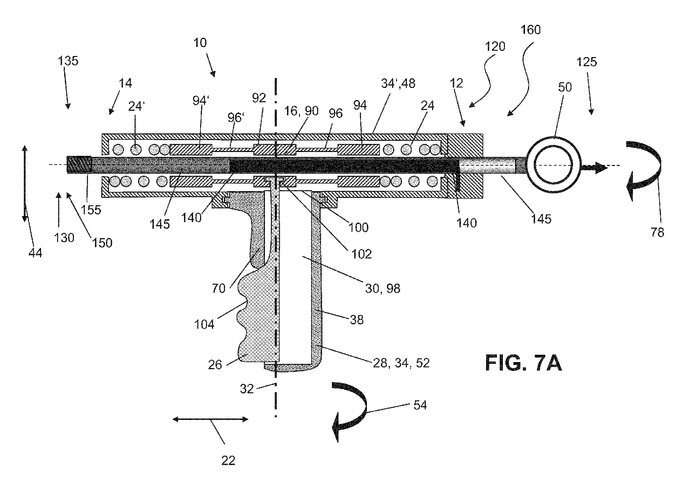

In den Fig. 7A und 7B ist ein dritter Betriebsmodus für eine schnelle Freigabe des Implantats 155, ein so genannter Fast-Release-Modus, gezeigt. Der Fast-Release-Modus wird durch Lösen der verliersicheren Verbindung zwischen der Buchse 92 und dem äußeren Einführelement 145 aktiviert. Dies ist nur bei einem lösbaren Kraft- und/oder Formschluss zwischen der Buchse 92 und dem äußeren Einführelement 145 möglich. Bei einer Ausgestaltung der Freigabevorrichtung 160 mit einer stoffschlüssigen Verbindung zwischen der Buchse 92 und dem äußeren Einführelement 145 ist ein Fast release-Modus nicht möglich. Das Lösen des Kraft- und/oder Formschlusses wäre alternativ zu der oben ausgeführten Variante mit dem Klemmmittel beispielsweise dadurch realisierbar, dass das äußere Einführelement 145 innerhalb des Körpers 10 über seine Kontur oder seinen Umfang in Umfangsrichtung 78 mit unterschiedlichen Breiten oder Durchmessern ausgeführt ist. Im Deploy -oder Retrieval-Mode besteht der Form- und/oder Kraftschluss zwischen der Buchse 92 und dem äußeren Einführelement 145 über eine größere Breite oder einem größeren Durchmesser des äußeren Einführelements 145. Werden nun der Körper 10 bzw. die Freigabevorrichtung 160 und die Einführelemente 140, 145 in Umfangsrichtung 78 des Körpers 10 um ca. 90° gegeneinander gedreht nimmt eine kleinere Breite oder ein kleinerer Durchmesser des äußeren Einführelements 145 die Position der größeren Breite oder des größeren Durchmessers ein (nicht im Detail gezeigt). Hierdurch löst sich der Form- und oder Kraftschluss zwischen der Buchse 92 und dem äußeren Einführelement 145, so dass das äußere Einführelement 145 relativ zum Kraftübertragungsbereich 90 frei bewegt werden kann. Die Endbereich 94, 94' des Übertragungselements 16 sind als Gleitbereiche 94, 94' ausgeführt, um eine Gleitbewegung des Übertragungselements 16 auf dem äußeren Einführelement 145 zu vermitteln.

Zu der Anzeige des nun eingestellten Betriebsmodus wird der Schriftzug 58 "FAST RELEASE" im Fenster 68 des Displays 60 angezeigt (vgl. Fig. 7B). Dies kann durch eine Blende erfolgen, die durch das Drehen des Körpers 10 in Umfangsrichtung 78 bewegt wird, wodurch der Schriftzug 58 im Fenster 68 erscheint. Nun kann der Bediener am Bedienelement 50 am proximalen Ende der äußeren Einführvorrichtung 145 zur schnellen Freigabe des Implantats 155 ziehen (siehe Pfeil Fig. 7A). Hierdurch bewegt sich das äußere Einführelement 145 in Richtung des proximalen Endes 12 und das Implantat 155 wird freigegeben (nicht gezeigt).

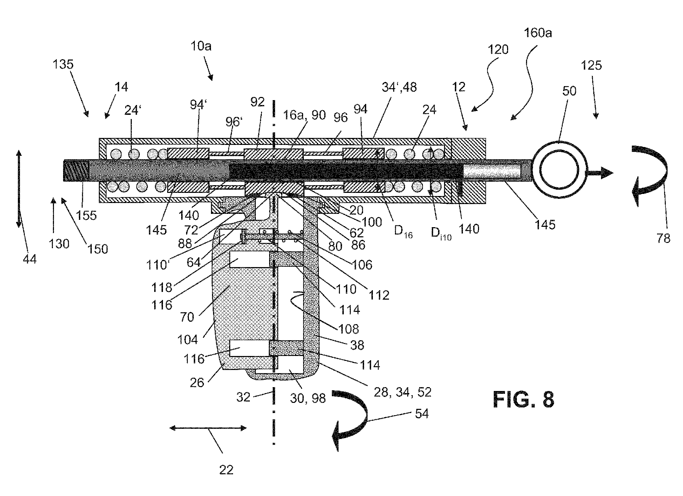

In Fig. 8 bis 12 sind zwei alternative Ausführungsbeispiele des Körpers 10 bzw. der Freigabevorrichtung 160 dargestellt. Im Wesentlichen sind gleich bleibende Bauteile, Merkmale und Funktionen grundsätzlich mit den gleichen Bezugszeichen beziffert. Zur Unterscheidung des Ausführungsbeispiels der Fig. 8 bis 12 zu dem in den Fig. 1 bis 7 ist jedoch den Bezugszeichen der in dem Ausführungsbeispiel der Fig. 8 bis 12 abweichenden ausgeführten Bauteile der Buchstabe a oder b hinzugefügt. Die nachfolgende Beschreibung beschränkt sich im Wesentlichen auf die Unterschiede zu dem Ausführungsbeispiel in den Fig. 1 bis 7, wobei bezüglich gleich bleibender Bauteile, Merkmale und Funktionen auf die Beschreibung des Ausführungsbeispiels in den Fig. 1 bis 7 verwiesen werden kann.

Die Freigabevorrichtung 160a bzw. der Körper 10a der Fig. 8 bis 11 unterscheidet sich von dem Körper 10 und der Freigabevorrichtung 160 der Fig. 1 bis 7 dadurch, dass ein Übertragungselement 16a mittels einer speziellen Ausgestaltung des Kraftübertragungsbereichs 90 einen lösbaren Kraftschluss zwischen dem Übertragungselement 16a und dem äußeren Einführelement 145 vermitteln kann. Das Übertragungselement 16a ist als eine sich koaxial zu den Einführelementen 140, 145 einer Einführvorrichtung 120 und sich in Umfangsrichtung 78 um den Körper 10a erstreckende Buchse 92 ausgeführt. Für die Vermittlung des Kraftschlusses ist der Kraftübertragungsbereich 90 mit zwei Schrägen 86, 88 ausgestattet, die von einem mittigen Bereich 64 in radialer Richtung 44 schräg nach außen verlaufen. Ein axial in Längsrichtung 22 im Körper 10a verschieblicher Aktor 26 weist an seinem radial oberen Ende eine Anlagefläche 80 auf, die in einem unaktuierten Zustand des Aktors 26 an dem Bereich 64 anliegt (vgl. Fig. 8). Zu einer Lagerung des Aktors 26 und zu dessen axialer Führung ist ein Schlitz 100 in einem Gehäuse 48 des Körpers 10a vorgesehen, der als eine Schiene dient (vgl. auch Fig. 2).

In der Fig. 8 ist eine Ausgangsposition der Freigabevorrichtung 160a für eine langsame Freigabe eines Implantats 155, dem Deploy-Modus, gezeigt. Hierbei sind alle Bauteile im Wesentlichen analog zu der Darstellung der Fig. 3A angeordnet. Wird nun der Aktor 26 betätigt bzw. in Richtung eines proximalen Endes 12 des Körpers 10a verschoben, bewegt sich die Anlagefläche 80 des Aktors 26 entlang der Schräge 86 in Richtung des proximalen Endes 12, wodurch der Kraftübertragungsbereich 90 in seiner Achse 20 entgegen dem Uhrzeigersinn gekippt wird.

Das Kippen des Kraftübertragungsbereichs 90 relativ zu Endbereichen 94, 94' der Buchse 92 ist möglich, da jeweils axial zwischen dem Kraftübertragungsbereich 90 und einem der Endbereiche 94, 94' ein Übergangsbereich 96, 96' angeordnet ist, der eine dünnere Materialstärke aufweist als der Kraftübertragungsbereich 90 und die Endbereiche 94, 94'. Dadurch weisen die Übergangsbereich 96, 96' eine geringerer Biegesteifigkeit auf als der Kraftübertragungsbereich 90 und die Endbereiche 94, 94', wodurch das Kippen des Kraftübertragungsbereichs 90 ohne dessen Übertragung auf die Endbereiche 94, 94' möglich wird. Bei dem Kippen des Kraftübertragungsbereichs 90 werden die Übergangsbereiche 96, 96' der Buchse 92 im Uhrzeigersinn gebogen. Das Übertragungselement 16a hat einen Durchmesser D₁₆ der so ausgewählt ist, dass das Übertragungselement 16 mit Spiel für eine Kippbewegung des Übertragungselements 16 zu einem Innendurchmesser Dᵢ₁₀ des Körpers 10a angeordnet ist (vgl. Fig. 8).

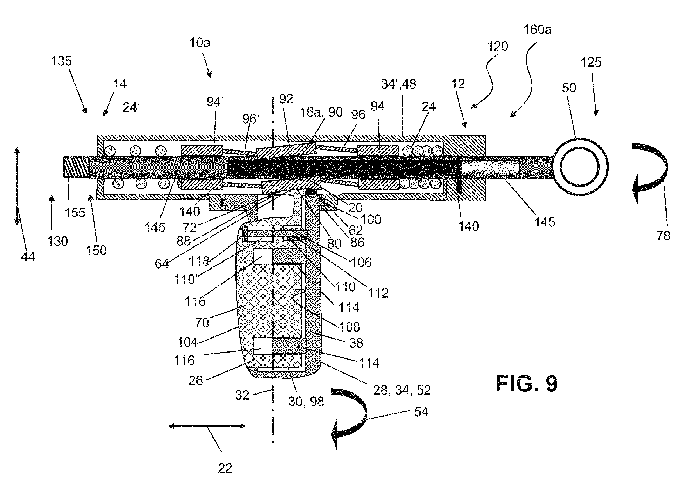

In dieser gekippten Position etabliert der Kraftübertragungsbereich 90 einen Kraftschluss mit dem äußeren Einführelement 145, wodurch das Übertragungselement 16a für die Freigabe des Implantats 155 entgegen des Uhrzeigersinns kippbar ist. Um nun das äußere Einführelement 145 in Richtung des proximalen Endes 12 zu bewegen, weist das Übertragungselement 16a einen Anschlag 62 für den Aktor 26 auf. Schlägt nun der Aktor 26 an den Anschlag 62 an, schiebt der bei seiner weiteren Bewegung in Richtung des proximalen Endes 12 den Kraftübertragungsbereich 90 in Richtung des proximalen Endes 12. Wegen des Kraftschlusses zwischen dem Kraftübertragungsbereich 90 und dem äußeren Einführelement 145, wird auch dieses in Richtung des proximalen Endes 12 geschoben. Hierbei wird eine Federelement 24, das axial zwischen dem Endbereich 94 der Buchse 92 und dem proximalen Ende 12 angeordnet ist, gestaucht (siehe Fig. 9).

Bei dem Verschieben des Aktors 26 in Richtung des proximalen Endes 12 des Körpers 10a, wird der Aktor 26 durch ein Federelement 106, beispielsweise in der Form einer Spiralfeder, vorgespannt. Dieses Federelements 106 erstreckt sich in seinem im Wesentlichen entspannten Zustand zwischen einer vertikalen Innenwand 108 des Griffsegments 34, die in Richtung der Aufnahme 98 weist, und einer vertikalen Wand einer proximalen Aussparung 110 im Aktor 26 (vgl. Fig. 8). Damit schlägt ein distales Ende des Federelements 106 an die Wand der proximalen Aussparung 110 an. Zu einer Lagerung des Federelements 106 ist dieses um ein Lagerelement 112 gelegt, welches an der Innenwand 108 des Griffsegments 34 angeordnet bzw. angeformt ist. Das Lagerelement 112 erstreckt sich im Wesentlichen parallel zu der Bewegungsrichtung des Aktors 26 und durchspannt die korrespondierende proximale Aussparung 110.

Zu einer homogenen axialen Führung des Aktors 26, weist die Innenwand 108 zwei Führungsstege 114 auf, die vertikal beabstandet und parallel zueinander sowie zur Bewegungsrichtung der Aktors 26 orientiert sind. Jeder Führungssteg 114 greift in eine korrespondierende Aussparung 116 des Aktors 26 ein. Wie in Fig. 9 zu sehen ist, wird die Bewegung des Aktors 26 in Richtung des proximalen Endes 12 des Körpers 10a durch ein Anschlagen eines vertikalen Endstegs 118 des Lagerelements 112 an eine distale Wand einer distalen Aussparung 110' des Aktors 26 limitiert. Um eine harmonische, abgestimmte und gezielte Bewegung des Aktors 26 zu gewährleisten sind die Orientierungen und Dimensionen, wie bspw. Länge, Breite etc., des Lagerelements112, der Führungsstege 114 und der Aussparungen 110, 110', 112 aufeinander angepasst.

Grundsätzlich könnte das Federelement 106 auch axial zwischen dem radial oberen Ende des Aktor 26 und dem Anschlag 62 eingespannt sein. Hierbei müsste für das Abdecken des Implantats 155 (siehe unten) ein zweites Federelement 106 vorgesehen werden, das axial zwischen dem radial oberen Ende des Aktor 26 und einem Anschlag 72 gespannt ist (nicht gezeigt).

Wird nun der Aktor 26 losgelassen, bewegt sich dieser durch eine Federkraft des Federelements 106 und geführt durch den Schlitz 100, zurück in seine Ausgangsposition (vgl. Fig. 8). Dadurch wird der Kraftschluss zwischen dem Kraftübertragungsbereich 90 und dem äußeren Einführelement 145 gelöst, wodurch dieser wieder seine koaxiale Position zu den Einführelementen 140, 145 einnehmen kann. Das Federelement 24, das auch eine Rückstellfeder ist, kann expandieren und drückt die Buchse 92 mit dem Kraftübertragungsbereich 90 in Längsrichtung 22 und in Richtung des distalen Endes 14 zurück in die mittlere Position bzw. die Ausgangsposition.

Somit vermittelt der Aktors 26 sowohl die gezielte Bewegung in Längsrichtung 22 des Übertragungselements 16a für die gezielte Relativbewegung in Längsrichtung 22 zwischen dem ersten und dem zweiten Einführelement 140, 145 wie auch eine Kippbewegung des Übertragungselements 16a bzw. es resultiert durch die Betätigung des Aktors 26 ein Zurückziehen des Außenschafts relativ zum Innenschaft und damit auch zum Implantat 155. Hierdurch wird das Implantat 155 freigelegt und expandiert (siehe Fig. 9). Im Anschluss wird die Freigabevorrichtung 160a bzw. der Innenschaft in den Außenschaft zurückgezogen und die Einführvorrichtung 120 aus dem Körper entfernt. Das Implantat 155 verbleibt vollständig positioniert im Körper (nicht gezeigt).

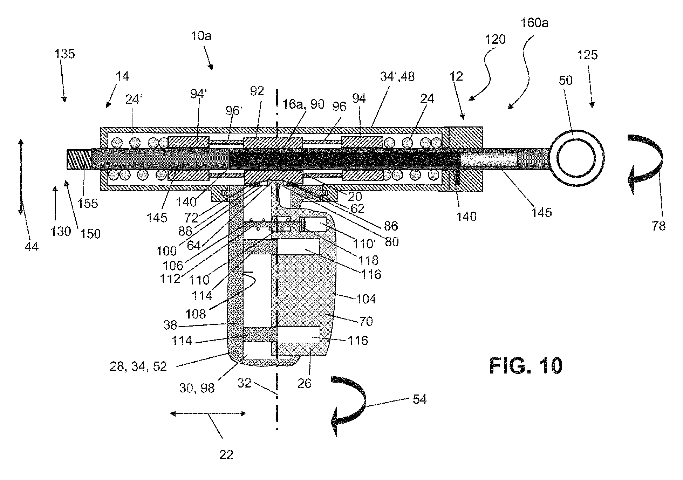

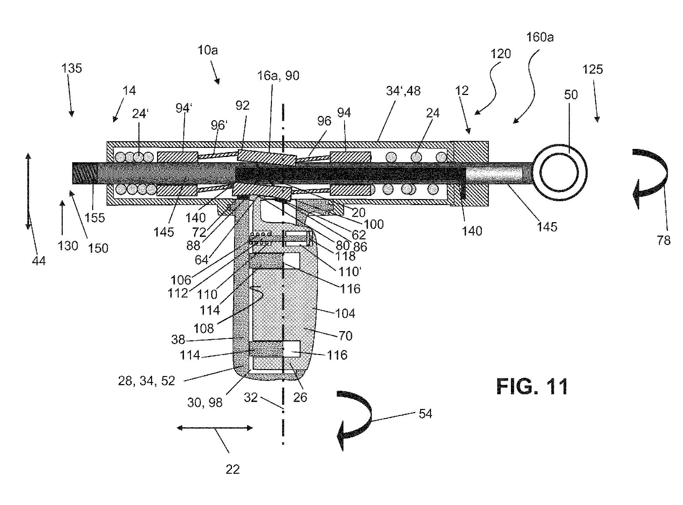

In der Fig. 10 ist eine Ausgangsposition der Freigabevorrichtung 160a für ein Abdecken des Implantats 155, dem Retrieval-Modus, gezeigt. Hierbei sind alle Bauteile im Wesentlichen analog zu der Darstellung der Fig. 5A angeordnet. Dieser Modus wird durch ein Drehen des ersten Griffsegments 34 um seine Drehachse 32 eingestellt. Wird nun der Aktor 26 betätigt bzw. in Richtung eines distalen Endes 14 des Körpers 10a verschoben, bewegt sich die Anlagefläche 80 des Aktors 26 entlang der Schräge 88 in Richtung des distalen Endes 14, wodurch der Kraftübertragungsbereich 90 in seiner Achse 20 im Uhrzeigersinn gekippt wird. In dieser gekippten Position etabliert der Kraftübertragungsbereich 90 einen Kraftschluss mit dem äußeren Einführelement 145, wodurch das Übertragungselement 16a für die Abdeckung des Implantats 155 im Uhrzeigersinn kippbar ist. Um nun das äußere Einführelement 145 in Richtung des distalen Endes 14 zu bewegen, weist das Übertragungselement 16a einen Anschlag 72 auf. Schlägt nun der Aktor 26 an den Anschlag 72 an und wird der Aktor 26 weiter in Richtung des distalen Endes 14 bewegt schiebt der Aktor 26 den Kraftübertragungsbereich 90 in Richtung des distalen Endes 14. Wegen des Kraftschlusses zwischen dem Kraftübertragungsbereich 90 und dem äußeren Einführelement 145, wird auch dieses in Richtung des distalen Endes 14 geschoben. Hierbei wird eine Federelement 24', das axial zwischen dem Endbereich 94' der Buchse 92 und dem distalen Ende 14 angeordnet ist, gestaucht (siehe Fig. 11). An einer Katheterspitze 150 ergibt sich also ein Vorschieben des äußeren Einführelements 145 zur Katheterspitze 150 hin. Wird das Implantat 155 durch das innere Einführelement 140 axial blockiert, schiebt sich das äußere Einführelement 145 also (wieder) über das Implantat 155 und bedeckt dieses.

Wird nun der Aktor 26 losgelassen bewegt sich dieser durch die Federkraft des Federelements 106 und geführt durch den Schlitz 100 zurück in seine Ausgangsposition. Dadurch wird der Kraftschluss zwischen dem Kraftübertragungsbereich 90 und dem äußeren Einführelement 145 gelöst kann der Kraftübertragungsbereich 90 wieder seine koaxiale Position zu den Einführelementen 140, 145 einnehmen kann. Das Federelement 24', das auch eine Rückstellfeder ist, kann expandieren und drückt die Buchse 92 mit dem Kraftübertragungsbereich 90 in Längsrichtung 22 und in Richtung des distalen Endes 14 zurück in die mittlere Position bzw. die Ausgangsposition.

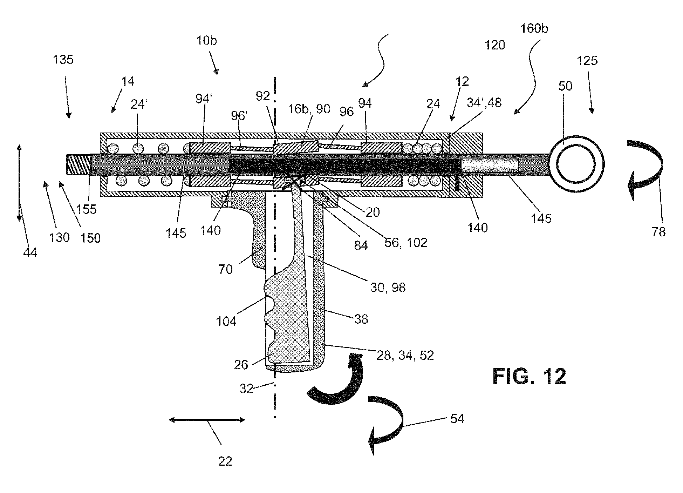

Die Freigabevorrichtung 160b bzw. der Körper 10b der Fig. 12 unterscheidet sich von dem Körper 10 und der Freigabevorrichtung 100 der Fig. 1 bis 7 dadurch, dass durch ein Kippen eines Aktors 26 zusammen mit einem Übertragungselement 16b und anhand eines Kraftübertragungsbereichs 90 ein lösbarer Kraftschluss zwischen dem Übertragungselement 16b und dem äußeren Einführelement 145 vermittelt wird.

Hierfür ist der axial verschieblichen Aktor 26 in der Form eines Bedienhebels im ersten Griffsegment 34 über einen Anlagepunkt 56 in einer Aufnahme 102 des Kraftübertragungsbereichs 90 schwenkbar gelagert. Der Anlagepunkt 56 ist eine Schwenkachse 84 des Aktors 26, die parallel zu einer Drehachse 20 des Übertragungselements 16b ausgerichtet ist, und die in einer Ausgangsposition des unaktuierten Aktors 26, bei der eine Buchse 92 mit dem Kraftübertragungsbereich 90, in einer mittleren Position in einem Körper 16b angeordnet ist, in derselben axialen Höhe wie die Drehachse 32 des ersten Griffsegments 34 liegt (nicht im Detailgezeigt, Anordnung analog zur Anordnung in Fig. 1).

Bei einer Ausgangsposition, die vor einer Implantation eines Implantats 155 eingestellt ist, ist der Kraftübertragungsbereich 90 in seiner Achse 20 axial zwischen zwei Federelementen 24, 24' fixiert und erstreckt sich koaxial zu Einführelementen 140, 145 einer Einführvorrichtung 120 (nicht im Detailgezeigt, Anordnung analog zur Anordnung in Fig. 1). Wird nun der Aktor 26 entgegen dem Uhrzeigersinn im Anlagepunkt 56 geschwenkt (siehe Pfeil in Fig. 12), wird das Übertragungselement 16b ebenso entgegen dem Uhrzeigersinn um seine Achse 20 gekippt. Dies wird aufgrund der verringerten Biegesteifigkeit von Übergangsbereichen 96, 96' der Buchse 92 und damit deren Biegung im Uhrzeigersinn ermöglicht. Der Aktor 26 bewirkt somit eine kraftschlüssige Verbindung mit dem äußeren Einführelement 145, wodurch dieses relativ zu dem Körper 10b und dem inneren Einführelement 140 axial fixiert ist. Wenn kein weiteres Kippen mehr möglich ist, verschiebt der Aktor 26 das gekippte Übertragungselement 16b bzw. die Buchse 92 zusammen mit dem äußeren Einführelement 145 axial in Richtung des proximalen Endes 12. Dies erfolgt, bis das Federelement 24 vollständig zusammengedrückt ist, was die axiale Bewegung des Aktors 26 limitiert (siehe Fig. 12).

Durch Loslassen des Aktors 26 bewegt sich dieser wieder zurück in seiner unaktuierte Position bzw. die Ausgangsposition, wodurch auch das Übertragungselement 16b in seine koaxiale Position zu den Einführelementen 140, 145 zurückkehrt (nicht gezeigt). Aufgrund dessen wird die kraftschlüssige Verbindung zwischen dem Kraftübertragungsbereich 90 und dem äußeren Einführelement 145 aufgelöst. Somit dehnt sich das Federelement 24, das als eine Rückstellfeder ausgeführt ist, wieder aus und die Buchse 92 wird in Längsrichtung 22 und in Richtung des distalen Endes 14 in die mittige Position oder die Ausgangsposition geschoben (nicht gezeigt).

Die Betätigung des Aktors 26 resultiert in ein Zurückziehen des Außenschafts relativ zum Innenschaft und damit auch zum Implantat 155. Hierdurch wird das Implantat 155 freigelegt und expandiert (siehe Fig. 12). Im Anschluss wird die Freigabevorrichtung 160b bzw. der Innenschaft in den Außenschaft zurückgezogen und die Einführvorrichtung 120 aus dem Körper entfernt. Das Implantat 155 verbleibt vollständig positioniert im Körper (nicht gezeigt).

Ein Retrieval Modus kann nach dem Drehen des ersten Griffsegments 34 um die Drehachse 32 erfolgen. Dies wird ausgelöst durch ein Schwenken des Aktors 26 und damit des Übertragungselements 16b im Uhrzeigersinn und läuft mit den Spezifikationen des in der Fig. 12 beschriebenen Ausführungsbeispiels im Wesentlichen analog zu dem Ablauf des in den Fig. 10 und 11 beschriebenen Ausführungsbeispiel ab, womit hier auf diese Beschreibung verwiesen wird.

**Bezugszeichen**

| | | | |
|---|---|---|---|
| 10 | Körper | 72 | Anschlag |
| 12 | Ende | 78 | Umfangsrichtung |
| 14 | Ende | 80 | Anlagefläche |
| 16 | Übertragungselement | 84 | Schwenkachse |
| 20 | Achse | 86 | Schräge |
| 22 | Längsrichtung | 88 | Schräge |
| 24 | Federelement | 90 | Kraftübertragungsbereich |
| 26 | Aktor | 92 | Buchse |
| 28 | Stellelement | 94 | Bereich |
| 30 | Wirkelement | 96 | Übergangsbereich |
| 32 | Drehachse | 98 | Ausnehmung |
| 34 | Griffsegment | 100 | Schlitz |
| 36 | Aufnahme | 102 | Aufnahme |
| 38 | Anzeigeelement | 104 | Bedienoberfläche |
| 40 | Durchführung | 106 | Federelement |
| 42 | Durchführung | 108 | Innenwand |
| 44 | radialer Richtung | 110 | Aussparung |
| 46 | Verankerung | 112 | Lagerelement |
| 48 | Gehäuse | 114 | Führungssteg |
| 50 | Bedienelement | 116 | Aussparung |
| 52 | Pistolengriff | 118 | Endsteg |
| 54 | Umfangsrichtung | 120 | Einführvorrichtung |
| 56 | Anlagepunkt | 125 | Ende |
| 58 | Schriftzug | 130 | Ende |
| 60 | Display | 135 | Schaftbereich |
| 62 | Anschlag | 140 | Einführelement |
| 64 | Bereich | 145 | Einführelement |
| 66 | Scheibe | 150 | Katheterspitze |
| 68 | Fenster | 155 | Implantat |
| 70 | Knauf | 160 | Freigabevorrichtung |
| D | Durchmesser | | |
| Di | Innendurchmesser | | |
| M | Materialstärke | | |

## Patentansprüche

1. Freigabevorrichtung (160, 160a, 160b) zum Lösen eines medizinischen Implantats (155) von einer Einführvorrichtung (120), bei welcher das Implantat (155) durch eine Relativbewegung zwischen einem ersten und einem zweiten Einführelement (140, 145) freisetzbar ist, umfassend einen Körper (10, 10a, 10b) mit einem proximalen Ende (12), das im Benutzungszustand von einem distalen Ende (130) der Einführvorrichtung (120) entfernt ist, und einem distalen Ende (14), das im Benutzungszustand dem distalen Ende (130) der Einführvorrichtung (120) zugewandt ist, wobei zwischen dem proximalen und dem distalen Ende (12, 14) ein erstes Griffsegment (34, 34'), ein zweites Griffsegment (34, 34') und zumindest ein Aktor (26) vorgesehen ist, wobei der zumindest eine Aktor (26) manuell bedienbar und im Wesentlichen axial verschieblich im Wesentlichen in zumindest das erste Griffsegment (34) bringbar ist, um eine gezielte Relativbewegung in Längsrichtung (22) zwischen dem ersten und dem zweiten Einführelement (140, 145) der Einführvorrichtung (120) zu bewirken.

2. Freigabevorrichtung nach Anspruch 1, wobei eine Drehachse (32) des ersten Griffsegments (34) im Wesentlichen senkrecht zu zumindest einem der Einführelemente (140, 145) verläuft und/oder das zumindest zweite Griffsegment (34') im Wesentlichen koaxial zu zumindest einem der Einführelemente (140, 145) verläuft.

3. Freigabevorrichtung nach Anspruch 1 oder 2, wobei die gezielte Relativbewegung in Längsrichtung (22) zwischen dem ersten und dem zweiten Einführelement (140, 145) der Einführvorrichtung (120) zumindest mittels eines Kraftschlusses, insbesondere eines lösbaren Kraftschlusses, zwischen dem zumindest einen Aktor (26) und einem der Einführelemente (145) erfolgt.

4. Freigabevorrichtung nach einem der vorhergehenden Ansprüche, aufweisend ein Stellelement (28), mit dem ein Betriebsmodus einstellbar und/oder anzeigbar ist, wobei bevorzugt zu einem Wechsel zwischen zumindest zwei Betriebsmodi das Stellelement (28) ein Wirkelement (30) aufweist, das insbesondere exzentrisch zu einer Drehachse (32) des Stellelements (28) angeordnet ist.

5. Freigabevorrichtung zumindest nach Anspruch 4, wobei das erste Griffsegment (34) einstückig mit dem Stellelement (28) ausgeführt ist.

6. Freigabevorrichtung nach einem der vorhergehenden Ansprüche, aufweisend ein Anzeigeelement (38) zu einer Anzeige eines eingestellten Betriebsmodus, wobei das Anzeigeelement (38) bevorzugt einstückig mit einem drehbaren Griffsegment (34) ausgeführt ist.

7. Freigabevorrichtung nach einem der vorhergehenden Ansprüche, aufweisend zumindest ein im Wesentlichen axial verschiebliches Übertragungselement (16, 16a, 16b), welches die axiale Bewegung des zumindest einen Aktors (26) beim Betätigen des zumindest einen Aktors (26) auf zumindest eines der Einführelemente (145) überträgt, um die gezielte Relativbewegung in Längsrichtung (22) zwischen dem ersten und dem zweiten Einführelement (140, 145) der Einführvorrichtung (120) zu bewirken.

8. Freigabevorrichtung nach Anspruch 7, wobei das zumindest eine Übertragungselement (16, 16a, 16b) eine zylindrische Buchse (92) aufweist, welche bevorzugt im Wesentlichen koaxial zu zumindest einem der Einführelemente (140, 145) angeordnet ist.

9. Freigabevorrichtung zumindest nach Anspruch 7, wobei das zumindest eine Übertragungselement (16, 16a, 16b) zumindest einen Kraftübertragungsbereich (90) und zumindest einen Gleitbereich (94, 94') aufweist, wobei bevorzugt der zumindest eine Gleitbereich (94, 94') eine Gleitbewegung des zumindest einen Übertragungselements (16, 16a, 16b) auf einem der Einführelemente (145) vermittelt.

10. Freigabevorrichtung nach Anspruch 9, wobei das zumindest eine Übertragungselement (16, 16a, 16b) zumindest einen Übergangsbereich (96, 96') umfasst, welcher bevorzugt axial zwischen dem zumindest einen Kraftübertragungsbereich (90) und dem zumindest einen Gleitbereich (94, 94') angeordnet ist und/oder wobei der zumindest eine Übergangsbereich (96, 96') und der zumindest eine Kraftübertragungsbereich (90) jeweils eine radiale Materialstärke (M₉₀, M₉₆) aufweisen, wobei insbesondere die Materialstärke (M₉₆) des zumindest einen Übergangsbereichs (96, 96') dünner ist als die Materialstärke (M₉₀) des zumindest einen Kraftübertragungsbereichs (90).

11. Freigabevorrichtung zumindest nach Anspruch 7, wobei das zumindest eine Übertragungselement (16, 16a, 16b) mit zumindest einem Federelement (24, 24') vorgespannt ist, wobei das zumindest eine Übertragungselement (16, 16a, 16b) durch das zumindest eine Federelement (24, 24') in seine Ausgangsposition zurückstellbar ist.

12. Freigabevorrichtung zumindest nach Anspruch 7, wobei für eine schnelle Freigabe des Implantats (155) das zumindest eine Übertragungselement (16, 16a, 16b) relativ zu zumindest einem der Einführelemente (145) im Wesentlichen koaxial stellbar ist.

13. Freigabevorrichtung nach einem der vorhergehenden Ansprüche, wobei eine axiale Bewegung des zumindest einen Aktors (26) in Richtung des proximalen Endes (12) des Körpers (10, 10a, 10b) eine Freigabe des Implantats (155) vermittelt und/oder eine axiale Bewegung des zumindest einen Aktors (26) in Richtung des distalen Endes (14) des Körpers (10, 10a, 10b) eine Abdeckung des Implantats (155) mit einem der Einführelemente (145) vermittelt.

14. Freigabevorrichtung nach einem der vorhergehenden Ansprüche, wobei der Körper (10, 10a, 10b) zumindest eine Aufnahme (36) für ein inneres Einführelement (140) zu einer unbeweglichen Fixierung des inneren Einführelements (140) am Körper (10, 10a, 10b) aufweist.

15. Freigabevorrichtung nach einem der vorhergehenden Ansprüche, wobei ein aus dem Körper (10, 10a, 10b) ragendes Einführelement (145) zumindest eine Markierung aufweist, die dazu vorgesehen ist anzuzeigen, wie viel vom Implantat (155) freigesetzt wurde, und/oder ob das Implantat (155) in die Freigabevorrichtung (160, 160a, 160b) zurückziehbar ist.

16. Einführvorrichtung (120) zum Einführen eines medizinischen Implantats (155), welches durch eine Relativbewegung zwischen einem ersten und einem zweiten Einführelement (140, 145) freisetzbar ist, umfassend eine Freigabevorrichtung (160, 160a, 160b) zum Lösen des medizinischen Implantats (155), insbesondere nach einem der Ansprüche 1 bis 15, umfassend einen Körper (10, 10a, 10b) mit einem proximalen Ende (12), das im Benutzungszustand von einem distalen Ende (130) der Einführvorrichtung (120) entfernt ist, und einem distalen Ende (14), das im Benutzungszustand dem distalen Ende (130) der Einführvorrichtung (120) zugewandt ist, wobei zwischen dem proximalen und dem distalen Ende (12, 14) ein erstes Griffsegment (34, 34'), ein zweites Griffsegment (34, 34') und zumindest ein Aktor (26) vorgesehen ist, wobei der zumindest eine Aktor (26) manuell bedienbar und im Wesentlichen axial verschieblich im Wesentlichen in zumindest das erste Griffsegment (34) bringbar ist, um eine gezielte Relativbewegung in Längsrichtung (22) zwischen dem ersten und dem zweiten Einführelement (140, 145) der Einführvorrichtung (120) zu bewirken.
